# EUROPEAN PATENT SPECIFICATION

(11) **EP 3 022 319 B1**
(45) Date of publication and mention of the grant of the patent: **17.05.2023**
(21) Application number: 14825773.6
(22) Date of filing: 14.07.2014
(51) Int. Cl.: C12Q 1/68, C12N 15/11, C12Q 1/6844, C12Q 1/6823

(54) **DETECTION OF TARGET NUCLEIC ACID SEQUENCE BY PTO CLEAVAGE AND EXTENSION-DEPENDENT IMMOBILIZED OLIGONUCLEOTIDE HYBRIDIZATION**
ERKENNUNG EINER NUKLEINSÄUREZIELSEQUENZ DURCH PTO-SPALTUNG UND ERWEITERUNGSABHÄNGIGE IMMOBILISIERTE OLIGONUKLEOTIDHYBRIDISIERUNG
DÉTECTION D'UNE SÉQUENCE CIBLE D'ACIDES NUCLÉIQUES PAR CLIVAGE PAR PTO ET HYBRIDATION D'OLIGONUCLÉOTIDES IMMOBILISÉS DÉPENDANT DE L'EXTENSION

(30) Priority: 15.07.2013 KR 20130083072
(43) Date of publication of application: 25.05.2016
(73) Proprietor: Seegene, Inc., Seoul 138-828 (KR)
(72) Inventor: CHUN, Jong Yoon, Seoul 135-957 (KR); LEE, Young Jo, Seoul 138-930 (KR)
(74) Representative: Scholz, Volker
(86) International application number: PCT/KR2014/006296
(87) International publication number: WO 2015/008985

(56) References cited:
- WO-A1-2012/096430
- WO-A1-2013/074163
- WO-A2-2007/011946
- WO-A2-2012/096523
- WO-A2-2012/134195
- WO-A2-2012/150835
- US-A- 5 691 142
- US-A1- 2004 023 271
- US-A1- 2006 040 282

## Description

### FIELD OF THE INVENTION

The present invention relates to a method and a kit for the detection of a target nucleic acid sequence in a nucleic acid sample by a PCE-IH (PTO Cleavage and Extension-Dependent Immobilized Oligonucleotide Hybridization) assay on a solid phase according to the appended set of claims.

### DESCRIPTION OF THE RELATED ART

DNA hybridization is a fundamental process in molecular biology. However, the conventional methods and processes depending mostly on hybridization are very likely to produce false positive results due to non-specific hybridization between probes and non-target sequences. Therefore, there remain problems to be solved for improving their reliability.

Besides probe hybridization processes, several approaches using additional enzymatic reactions, for example, TaqMan^{™} probe method, have been suggested.

In TaqMan^{™} probe method, the labeled probe hybridized with a target nucleic acid sequence is cleaved by a 5' nuclease activity of an upstream primer-dependent DNA polymerase, generating a signal indicating the presence of a target sequence (U.S. Pat. Nos. 5,210,015, 5,538,848 and 6,326,145). TheTaqMan^{™} probe method suggests two approaches for signal generation: polymerization-dependent cleavage and polymerization-independent cleavage. In polymerization-dependent cleavage, extension of the upstream primer must occur before a nucleic acid polymerase encounters the 5'-end of the labeled probe. As the extension reaction continues, the polymerase progressively cleaves the 5'-end of the labeled probe. In polymerization-independent cleavage, the upstream primer and the labeled probe are hybridized with a target nucleic acid sequence in close proximity such that binding of the nucleic acid polymerase to the 3'-end of the upstream primer puts it in contact with the 5'-end of the labeled probe to release the label. In addition, the TaqMan^{™} probe method discloses that the labeled probe at its 5'-end having a 5'-tail region not-hybridizable with a target sequence is also cleaved to form a fragment comprising the 5'-tail region.

There have been reported some methods in which a probe having a 5'-tail region non-complementary to a target sequence is cleaved by 5' nuclease to release a fragment comprising the S'-tail region.

For instance, U.S. Pat. No. 5,691,142 discloses a cleavage structure to be digested by 5' nuclease activity of DNA polymerase. The cleavage structure is exemplified in which an oligonucleotide comprising a 5' portion non-complementary to and a 3' portion complementary to a template is hybridized with the template and an upstream oligonucleotide is hybridized with the template in close proximity. The cleavage structure is cleaved by DNA polymerase having 5' nuclease activity or modified DNA polymerase with reduced synthetic activity to release the 5' portion non-complementary to the template. The released 5' portion is then hybridized with an oligonucleotide having a hairpin structure to form a cleavage structure, thereby inducing progressive cleavage reactions to detect a target sequence.

WO2012/096430A1 relates to the detection of a target nucleic acid sequence by a PTOCE (PTO Cleavage and Extension) assay. It discloses the detection of a target nucleic acid sequence in which the PTO (Probing and Tagging Oligonucleotide) hybridized with the target nucleic acid sequence is cleaved to release a fragment and the fragment is hybridized with the CTO (Capturing and Templating Oligonucleotide) to form an extended duplex, followed by detecting the presence of the extended duplex. The extended duplex provides signals (generation, increase, extinguishment or decrease of signals) from labels indicating the presence of the extended duplex and has adjustable Tm value, which are well adoptable for detection of the presence of the target nucleic acid sequence.U.S. Pat. No. 7,381,532 discloses a process in which the cleavage structure having the upstream oligonucleotide with blocked 3'-end is cleaved by DNA polymerase having 5' nuclease activity or FEN nuclease to release non-complementary 5' flap region and the released 5' flap region is detected by size analysis or interactive dual label. U.S. Pat. No. 6,893,819 discloses that detectable released flaps are produced by a nucleic acid synthesis dependent, flap-mediated sequential amplification method. In this method, a released flap from a first cleavage structure cleaves, in a nucleic acid synthesis dependent manner, a second cleavage structure to release a flap from the second cleavage structure and the release flaps are detected.

U.S. Pat. No. 7,309,573 disclose a method including formation of a released flap produced by a nucleic acid synthesis; extension of the released flap; cleavage of an oligonucleotide during extension of the flap and detection of a signal generated by the cleavage of the oligonucleotide.

By hybridization of fluorescence-labeled probes in a liquid phase, a plurality of target nucleic acid sequences may be simultaneously detected using even a single type of a fluorescent label by melting curve analysis. However, the conventional technologies for detection of target sequences by 5' nuclease-mediated cleavage of interactive-dual labeled probes require different types of fluorescent labels for different target sequences in multiplex target detection, which limits the number of target sequences to be detected due to limitation of the number of types of fluorescent labels.

U.S. Pat. Appln. Pub. 2008-0241838 discloses a target detection method using cleavage of a probe having a 5' portion non-complementary to a target nucleic acid sequence and hybridization of a capture probe. A label is positioned on the non-complementary 5' portion. The labeled probe hybridized with the target sequence is cleaved to release a fragment, after which the fragment is then hybridized with the capture probe to detect the presence of the target sequence. In this method, it is necessary that an uncleaved/intact probe is not hybridized with the capture probe. For that, the capture probe having a shorter length has to be immobilized onto a solid substrate. However, such a limitation results in lower efficiency of hybridization on a solid substrate and also in difficulties in optimization of reaction conditions.

Therefore, there remain long-felt needs in the art to develop novel approaches for detection of a target sequence, particularly multiple target sequences on a solid phase by not only hybridization but also enzymatic reactions such as 5' nucleolytic reaction with enhanced accuracy in more convenient, reliable and reproducible hybridization manner.

### SUMMARY OF THE INVENTION

The present inventors have made intensive researches to develop novel approaches to detect target sequences with more improved accuracy and convenience, inter alia, in a multiplex manner. As a result, we have established novel protocols for detection of target sequences, in which target detection is accomplished by probe hybridization, enzymatic reactions including 5' nucleolytic reaction and extension, and extension-dependent immobilized oligonucleotide hybridization. The present protocols ensure detection of multiple target sequences on a solid phase with more improved accuracy and convenience.

Accordingly, it is an object of this invention to provide a method for detecting a target nucleic acid sequence in a nucleic acid sample by a PCE-IH (PTO Cleavage and Extension-Dependent Immobilized Oligonucleotide Hybridization) assay on a solid phase.

It is another object of this invention to provide a kit for detecting a target nucleic acid sequence in a nucleic acid sample by a PCE-IH (PTO Cleavage and Extension-Dependent Immobilized Oligonucleotide Hybridization) assay on a solid phase.

Other objects and advantages of the present invention will become apparent from the detailed description to follow taken in conjugation with the appended claims and drawings.

### BRIEF DESCRIPTION OF THE DRAWINGS

Figs. 1A-1B show the schematic structures of PTO (Probing and Tagging Oligonucleotide), CTO (Capturing and Templating Oligonucleotide) and IO (immobilized oligonucleotide) used in the PCE-IH assay. Particularly, the 3'-ends of the PTO, CTO and IO are blocked to prohibit their extension.
Fig. 2 represents schematically an embodiment of the PCE-IH assay using a single label linked to the fragment released from the PTO. The IO comprises a sequence complementary to the extended sequence of the extended strand.
Fig. 3 represents schematically an embodiment of the PCE-IH assay using a label to be incorporated into the extended strand.
Fig. 4 shows the results of the PCE-IH assay using a single label linked to the 5'-end of the PTO for analyzing the presence of the genomic DNA of Neisseria gonorrhoeae. Where the target nucleic acid sequence was present, a fluorescent signal was detected on a solid substrate.
Figs. 5A-5B show the results for effects of the CTO on hybridization of the IO with the extended strand.
Figs. 6A-6B show the results of the PCE-IH assay with varying the mole ratios of the PTO to the CTO.

### DETAILED DESCRIPTION OF THIS INVETNION

In a first aspect of the present invention, there is provided a method for detecting a target nucleic acid sequence in a nucleic acid sample by a PCE-IH (PTO Cleavage and Extension-Dependent Immobilized Oligonucleotide Hybridization) assay on a solid phase, comprising:
(a) hybridizing the target nucleic acid sequence with a PTO (Probing and Tagging Oligonucleotide); wherein the PTO comprises (i) a 3-targeting portion comprising a hybridizing nucleotide sequence complementary to the target nucleic acid sequence and (ii) a 5'-tagging portion comprising a nucleotide sequence non-complementary to the target nucleic acid sequence; wherein the 3-targeting portion is hybridized with the target nucleic acid sequence and the 5'-tagging portion is not hybridized with the target nucleic acid sequence;
(b) contacting the resultant of the step (a) to the enzyme having a 5' nuclease activity under conditions for cleavage of the PTO;
   wherein the PTO is cleaved by the enzyme having the 5' nuclease activity such that the cleavage releases a fragment comprising the 5'-tagging portion or a part of the 5'-tagging portion of the PTO;
(c) hybridizing the fragment released from the PTO with a CTO (Capturing and Templating Oligonucleotide); wherein the CTO comprises in a 3' to 5' direction (i) a capturing portion comprising a nucleotide sequence complementary to the 5'-tagging portion or a part of the 5'-tagging portion of the PTO and (ii) a templating portion comprising a nucleotide sequence non-complementary to the 5'-tagging portion and the 3'-targeting portion of the PTO; wherein the fragment released from the PTO is hybridized with the capturing portion of the CTO;
(d) performing an extension reaction using the resultant of the step (c) and a template-dependent nucleic acid polymerase; wherein the fragment hybridized with the capturing portion of the CTO is extended to produce an extended strand containing an extended sequence complementary to the templating portion of the CTO and an extended duplex is formed; wherein the extended strand comprises a label;
(e) hybridizing the extended strand with an IO (immobilized oligonucleotide) immobilized on a solid substrate; wherein the IO comprises a sequence complementary to the extended strand; wherein the hybridization between the extended strand with the IO provides a detectable signal on the solid substrate; wherein the sequence of the IO is selected not to form a hybrid with an undigested PTO and
(f) detecting the signal indicative of the presence of the extended strand; the presence of the extended strand indicates the presence of the target nucleic acid sequence.

The present inventors have made intensive researches to develop novel approaches to detect target sequences with more improved accuracy and convenience, inter alia, in a multiplex manner. As a result, we have established novel protocols for detection of target sequences, in which target detection is accomplished by probe hybridization, enzymatic reactions including 5' nucleolytic reaction and extension, and extension-dependent immobilized oligonucleotide hybridization. The present protocols ensure detection of multiple target sequences on a solid phase with more improved accuracy and convenience.

The present invention employs successive events including PTO (Probing and Tagging Oligonucleotide) hybridization; cleavage of the PTO and extension; and extension-dependent immobilized oligonucleotide hybridization. Therefore, it is named as a PCE-IH (PTO Cleavage and Extension-Dependent Immobilized Oligonucleotide Hybridization) assay.

The present invention will be described in more detail as follows:

### Step (a): Hybridization of an upstream oligonucleotide and a PTO with a target nucleic acid sequence

According to the present invention, a target nucleic acid sequence is first hybridized with an upstream oligonucleotide and a PTO (Probing and Tagging Oligonucleotide).

The term used herein "target nucleic acid", "target nucleic acid sequence" or "target sequence" refers to a nucleic acid sequence of interest for detection, which is annealed to or hybridized with a probe or primer under hybridization, annealing or amplifying conditions.

The term used herein "probe" refers to a single-stranded nucleic acid molecule comprising a portion or portions that are substantially complementary to a target nucleic acid sequence.

The term "primer" as used herein refers to an oligonucleotide, which is capable of acting as a point of initiation of synthesis when placed under conditions in which synthesis of primer extension product which is complementary to a nucleic acid strand (template) is induced, i.e., in the presence of nucleotides and an agent for polymerization, such as DNA polymerase, and at a suitable temperature and pH.

In a certain embodiment, the probe and primer are single-stranded deoxyribonucleotide molecules. The probes or primers used in this invention may be comprised of naturally occurring dNMP (i.e., dAMP, dGM, dCMP and dTMP), modified nucleotide, or non-natural nucleotide. The probes or primers may also include ribonucleotides.

The primer must be sufficiently long to prime the synthesis of extension products in the presence of the agent for polymerization. The exact length of the primers will depend on many factors, including temperature, application, and source of primer. The term "annealing" or "priming" as used herein refers to the apposition of an oligodeoxynucleotide or nucleic acid to a template nucleic acid, whereby the apposition enables the polymerase to polymerize nucleotides into a nucleic acid molecule which is complementary to the template nucleic acid or a portion thereof.

The term used "hybridizing" used herein refers to the formation of a double-stranded nucleic acid from complementary single stranded nucleic acids. The hybridization may occur between two nucleic acid strands perfectly matched or substantially matched with some mismatches. The complementarity for hybridization may depend on hybridization conditions, particularly temperature.

The hybridization of a target nucleic acid sequence with the upstream oligonucleotide and the PTO may be carried out under suitable hybridization conditions routinely determined by optimization procedures. Conditions such as temperature, concentration of components, hybridization and washing times, buffercomponents, and their pH and ionic strength may be varied depending on various factors, including the length and GC content of oligonucleotide (upstream oligonucleotide and PTO) and the target nucleotide sequence. For instance, when a relatively short oligonucleotide is used, it is suitable that low stringent conditions are adopted. The detailed conditions for hybridization can be found in Joseph Sambrook, et al., Molecular Cloning, A Laboratory Manual, Cold Spring Harbor Laboratory Press, Cold Spring Harbor, N.Y.(2001); and M.L.M. Anderson, Nucleic Acid Hybridization, Springer-Verlag New York Inc. N.Y.(1999).

There is no intended distinction between the terms "annealing" and "hybridizing", and these terms will be used interchangeably.

The upstream oligonucleotide and PTO have hybridizing nucleotide sequences complementary to the target nucleic acid sequence. The term "complementary" is used herein to mean that primers or probes are sufficiently complementary to hybridize selectively to a target nucleic acid sequence under the designated annealing conditions or stringent conditions, encompassing the terms "substantially complementary" and "perfectly complementary", for instance, perfectly complementary.

The 5'-tagging portion of the PTO comprises a nucleotide sequence non-complementary to the target nucleic acid sequence. The term "non-complementary" is used herein to mean that primers or probes are sufficiently non-complementary not to hybridize selectively to a target nucleic acid sequence under the designated annealing conditions or stringent conditions, encompassing the terms "substantially non-complementary" and "perfectly non-complementary", for instance, perfectly non-complementary.

For example, the term "non-complementary" in conjunction with the 5'-tagging portion of the PTO means that the 5'-tagging portion is sufficiently non-complementary not to hybridize selectively to a target nucleic acid sequence under the designated annealing conditions or stringent conditions, encompassing the terms "substantially non-complementary" and "perfectly non-complementary", for instance, perfectly non-complementary.

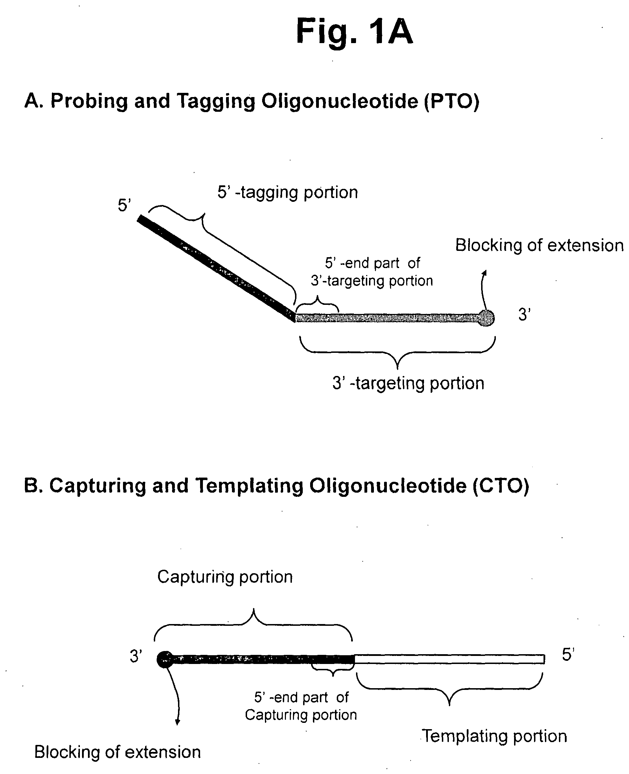

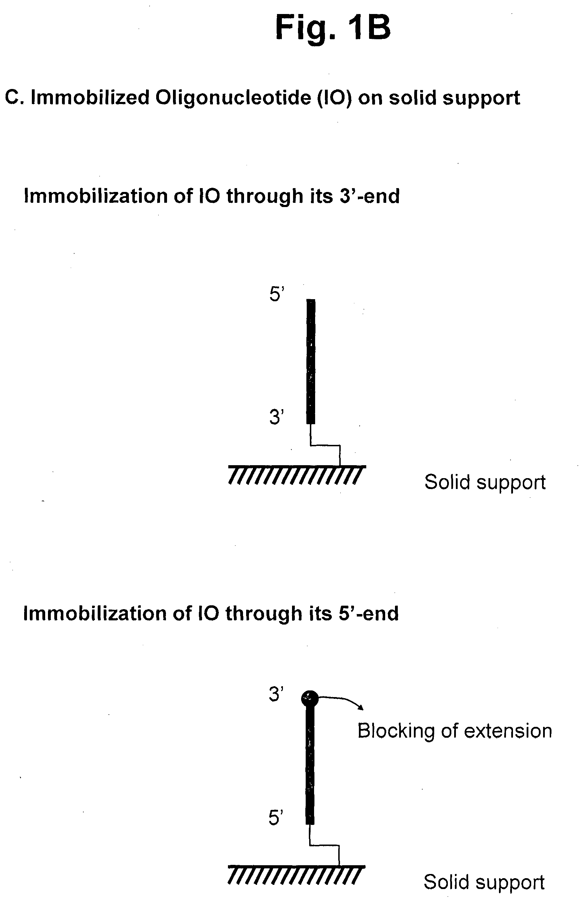

The term used herein "PTO (Probing and Tagging Oligonucleotide)" means an oligonucleotide comprising (i) a 3-targeting portion serving as a probe and (ii) a 5'-tagging portion with a nucleotide sequence non-complementary to the target nucleic acid sequence, which is nucleolytically released from the PTO after hybridization with the target nucleic acid sequence. The 5'-tagging portion and the 3-targeting portion in the PTO have to be positioned in a 5'to 3' order. The PTO is schematically illustrated in Fig. 1.

In an embodiment, the hybridization in step (a) is preformed under stringent conditions that the 3-targeting portion is hybridized with the target nucleic acid sequence and the 5'-tagging portion is not hybridized with the target nucleic acid sequence.

The PTO does not require any specific lengths. For example, the length of the PTO may be 15-150 nucleotides, 15-100 nucleotides, 15-80 nucleotides, 15-60 nucleotides, 15-40 nucleotides, 20-150 nucleotides, 20-100 nucleotides, 20-80 nucleotides, 20-60 nucleotides, 20-50 nucleotides, 30-150 nucleotides, 30-100 nucleotides, 30-80 nucleotides, 30-60 nucleotides, 30-50 nucleotides, 35-100 nucleotides, 35-80 nucleotides, 35-60 nucleotides, or 35-50 nucleotides. The 3'-targeting portion of the PTO may be in any lengths so long as it is specifically hybridized with target nucleic acid sequences. For example, the 3'-targeting portion of the PTO may be 10-100 nucleotides, 10-80 nucleotides, 10-50 nucleotides, 10-40 nucleotides, 10-30 nucleotides, 15-100 nucleotides, 15-80 nucleotides, 15-50 nucleotides, 15-40 nucleotides, 15-30 nucleotides, 20-100 nucleotides, 20-80 nucleotides, 20-50 nucleotides, 20-40 nucleotides or 20-30 nucleotides in length. The 5'-tagging portion may be in any lengths so long as it is specifically hybridized with the capturing portion of the CTO and then extended. For instance, the 5'-tagging portion of the PTO may be 5-50 nucleotides, 5-40 nucleotides, 5-30 nucleotides, 5-20 nucleotides, 10-50 nucleotides, 10-40 nucleotides, 10-30 nucleotides, 10-20 nucleotides, 15-50 nucleotides, 15-40 nucleotides, 15-30 nucleotides or 15-20 nucleotides in length.

The 3'-end of the PTO may have a 3'-OH terminal. In certain embodiment, the 3'-end of the PTO is "blocked" to prohibit its extension.

The blocking may be achieved in accordance with conventional methods. For instance, the blocking may be performed by adding to the 3'-hydroxyl group of the last nucleotide a chemical moiety such as biotin, labels, a phosphate group, alkyl group, non-nucleotide linker, phosphorothioate or alkane-diol. Alternatively, the blocking may be carried out by removing the 3'-hydroxyl group of the last nucleotide or using a nucleotide with no 3'-hydroxyl group such as dideoxynucleotide.

Alternatively, the PTO may be designed to have a hairpin structure.

The non-hybridization between the 5'-tagging portion of the PTO and the target nucleic acid sequence refers to non-formation of a stable double-strand between them under certain hybridization conditions. According to an embodiment of this invention, the 5'-tagging portion of the PTO not involved in the hybridization with the target nucleic acid sequence forms a single-strand.

The upstream oligonucleotide is located upstream of the PTO.

In addition, the upstream oligonucleotide or its extended strand hybridized with the target nucleic acid sequence induces cleavage of the PTO by an enzyme having a 5' nuclease activity.

The induction of the PTO cleavage by the upstream oligonucleotide may be accomplished by two fashions: (i) upstream oligonucleotide extension-independent cleavage induction; and (ii) upstream oligonucleotide extension-dependent cleavage induction.

Where the upstream oligonucleotide is positioned adjacently to the PTO sufficient to induce the PTO cleavage by an enzyme having a 5' nuclease activity, the enzyme bound to the upstream oligonucleotide digests the PTO with no extension reaction. In contrast, where the upstream oligonucleotide is positioned distantly to the PTO, an enzyme having a polymerase activity (e.g., template-dependent polymerase) catalyzes extension of the upstream oligonucleotide (e.g., upstream primer) and an enzyme having a 5' nuclease activity bound to the extended product digests the PTO.

Therefore, the upstream oligonucleotide may be located relatively to the PTO in two fashions. The upstream oligonucleotide may be located adjacently to the PTO sufficient to induce the PTO cleavage in an extension-independent manner. Alternatively, the upstream oligonucleotide may be located distantly to the PTO sufficient to induce the PTO cleavage in an extension-dependent manner.

The term used herein "adjacent" with referring to positions or locations means that the upstream oligonucleotide is located adjacently to the 3-targeting portion of the PTO to form a nick. Also, the term means that the upstream oligonucleotide is located 1-30 nucleotides, 1-20 nucleotides or 1-15 nucleotides apart from the 3'-targeting portion of the PTO.

The term used herein "distant" with referring to positions or locations includes any positions or locations sufficient to ensure extension reactions.

According to an embodiment, the upstream oligonucleotide is located distantly to the PTO sufficient to induce the PTO cleavage in an extension-dependent manner.

According to an embodiment, the upstream oligonucleotide is an upstream primer or an upstream probe. The upstream primer is suitable in an extension-independent cleavage induction or an extension-dependent cleavage, and the upstream probe is suitable in an extension-independent cleavage induction.

Alternatively, the upstream oligonucleotide may have a partial-overlapped sequence with the 5'-part of the 3'-targeting portion of the PTO. In certain embodiment, the overlapped sequence is 1-10 nucleotides, 1-5 nucleotides or 1-3 nucleotides in length. Where the upstream oligonucleotide has a partial-overlapped sequence with the 5'-part of the 3'-targeting portion of the PTO, the 3-targeting portion is partially digested along with the 5'-taggging portion in the cleavage reaction of the step (b). In addition, the overlapped sequence permits to cleave a desired site of the 3-targeting portion.

According to an embodiment, the upstream primer induces through its extended strand the cleavage of the PTO by the enzyme having the 5' nuclease activity.

The conventional technologies for cleavage reactions by upstream oligonucleotides may be applied to the present invention, so long as the upstream oligonucleotide induces cleavage of the PTO hybridized with the target nucleic acid sequence to release a fragment comprising the 5'-tagging portion or a part of the 5'-tagging portion of the PTO. For example, U.S. Pat. Nos. 5,210,015, 5,487,972, 5,691,142, 5,994,069 and 7,381,532 and U.S. Appln. Pub. No. 2008-0241838 may be applied to the present invention.

According to an embodiment, the method is performed in the presence of a downstream primer. The downstream primer generates additionally a target nucleic acid sequence to be hybridized with the PTO, enhancing sensitivity in target detection.

According to an embodiment, when the upstream primer and the downstream primer are used, a template-dependent nucleic acid polymerase is additionally employed for extension of the primers.

According to an embodiment, the upstream oligonucleotide (upstream primer or upstream probe), the downstream primer and/or 5'-tagging portion of the PTO have a dual priming oligonucleotide (DPO) structure developed by the present inventor. The oligonucleotides having the DPO structure show significantly improved target specificity compared with conventional primers and probes (see WO 2006/095981; Chun et al., Dual priming oligonucleotide system for the multiplex detection of respiratory viruses and SNP genotyping of CYP2C19 gene, Nucleic Acid Research, 35:6e40(2007)).

According to an embodiment, the 3-targeting portion of the PTO has a modified dual specificity oligonucleotide (mDSO) structure developed by the present inventor. The modified dual specificity oligonucleotide (mDSO) structure shows significantly improved target specificity compared with conventional probes (see WO 2011/028041).

### Step (b): Release of a fragment from the PTO cleavage

Afterwards, the resultant of the step (a) is contacted to an enzyme having a 5' nuclease activity under conditions for cleavage of the PTO. The PTO hybridized with the target nucleic acid sequence is digested by the enzyme having the 5' nuclease activity to release a fragment comprising the 5'-tagging portion or a part of the 5'-tagging portion of the PTO.

The term used herein "conditions for cleavage of the PTO" means conditions sufficient to digest the PTO hybridized with the target nucleic acid sequence by the enzyme having the 5' nuclease activity, such as temperature, pH, ionic strength, buffer, length and sequence of oligonucleotides and enzymes. For example, when Taq DNA polymerase is used as the enzyme having the 5' nuclease activity, the conditions for cleavage of the PTO include Tris-HCl buffer, KCl, MgCl₂ and temperature.

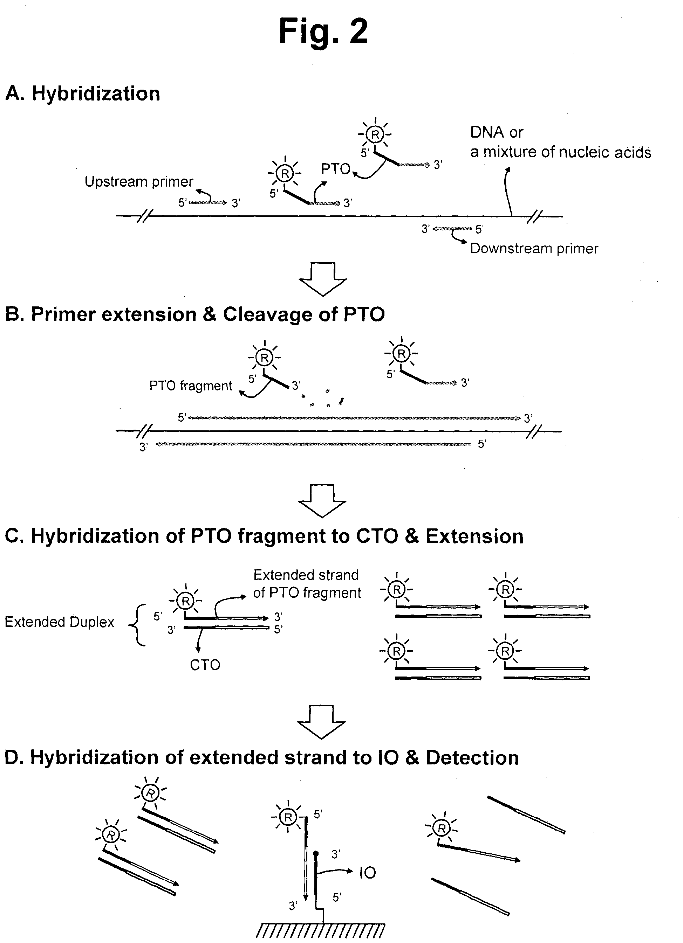

When the PTO is hybridized with the target nucleic acid sequence, its 3-targeting portion is involved in the hybridization and the 5'-tagging portion forms a single-strand with no hybridization with the target nucleic acid sequence (see Fig. 2). As such, an oligonucleotide comprising both single-stranded and double-stranded structures may be digested using an enzyme having a 5' nuclease activity by a variety of technologies known to one of skill in the art.

The cleavage sites of the PTO are varied depending on the type of upstream oligonucleotides (upstream probe or upstream primer), hybridization sites of upstream oligonucleotides and cleavage conditions (see U.S. Pat. Nos. 5,210,015, 5,487,972, 5,691,142, 5,994,069 and 7,381,532 and U.S. Appln. Pub. No. 2008-0241838).

A multitude of conventional technologies may be employed for the cleavage reaction of the PTO, releasing a fragment comprising the 5'-tagging portion or a part of the 5'-tagging portion.

Briefly, there may be three sites of cleavage in the step (b). Firstly, the cleavage site is a junction site between a hybridization portion of the PTO (3'-targeting portion) and a non-hybridization portion (5'-tagging portion). The second cleavage site is a site located several nucleotides in a 3'-direction apart from the 3'-end of the 5'-tagging portion of the PTO. The second cleavage site is located at the 5'-end part of the 3'-targeting portion of the PTO. The third cleavage site is a site located several nucleotides in a 5'-direction apart from the 3'-end of the 5'-tagging portion of the PTO.

According to an embodiment, the initial site for the cleavage of the PTO by the template-dependent polymerase having the 5' nuclease activity upon extension of the upstream primer is a starting point of the double strand between the PTO and the target nucleic acid sequence or a site 1-3 nucleotides apart from the starting point.

In this regard, the term used herein "a fragment comprising the 5'-tagging portion or a part of the 5'-tagging portion of the PTO" in conjunction with cleavage of the PTO by the enzyme having the 5' nuclease activity is used to encompass (i) the 5'-tagging portion, (ii) the 5'-tagging portion and the 5'-end part of the 3'-targeting portion and (iii) a part of the 5'-tagging portion. In this application, the term "a fragment comprising the 5'-tagging portion or a part of the 5'-tagging portion of the PTO" may be also described as "fragment" or "PTO fragment".

According to an embodiment, the PTO has a blocker portion containing a blocker resistant to cleavage by the enzyme having 5' nuclease activity and the blocker portion is used to control an initial cleavage site and/or successive cleavages. According to an embodiment, the PTO has a blocker portion containing as a blocker at least one nucleotide resistant to cleavage by the enzyme having 5' nuclease activity. For example, to induce cleavage at the junction site between a hybridization portion of the PTO (3'-targeting portion) and a non-hybridization portion (5'-tagging portion), the 5'-end part of 3'-targeting portion of PTO may be blocked with blockers. The number of blockers contained in the blocker portion may be not limited, including 1-10, 2-10, 3-8 or 3-6 blockers. The blockers present in the PTO may be in a continuous or intermittent manner, suitably a continuous manner. The nucleotides as blockers with a backbone resistant to the 5' to 3' exonuclease activity include any one known to one of skill in the art. For example, it includes various phosphorothioate linkages, phosphonate linkages, phosphoroamidate linkages and 2'-carbohydrates modifications. According to an embodiment, nucleotides having a backbone resistant to the 5' to 3' exonuclease activity include phosphorothioate linkage, alkyl phosphotriester linkage, aryl phosphotriester linkage, alkyl phosphonate linkage, aryl phosphonate linkage, hydrogen phosphonate linkage, alkyl phosphoroamidate linkage, aryl phosphoroamidate linkage, phosphoroselenate linkage, 2'-O-aminopropyl modification, 2'-O-alkyl modification, 2'-O-allyl modification, 2'-O-butyl modification, o-anomeric oligodeoxynucleotide and 1-(4'-thio-β-D-ribofuranosyl) modification.

According to an embodiment, a nucleotide as a blocker includes LNA (locked nucleic acid).

The term "part" used in conjunction with the PTO or CTO such as the part of the 5'-tagging portion of the PTO, the 5'-end part of the 3'-targeting portion of the PTO and the 5'-end part of the capturing portion of the CTO refers to a nucleotide sequence composed of 1-40, 1-30, 1-20, 1-15, 1-10 or 1-5 nucleotides, suitably 1, 2, 3 or 4 nucleotides.

According to an embodiment, the enzyme having the 5' nuclease activity is DNA polymerase having a 5' nuclease activity or FEN nuclease, suitably a thermostable DNA polymerase having a 5' nuclease activity or FEN nuclease.

A suitable DNA polymerase having a 5' nuclease activity in this invention is a thermostable DNA polymerase obtained from a variety of bacterial species, including Thermus aquaticus (Taq), Thermus thermophilus (Tth), Thermus filiformis, Thermis flavus, Thermococcus literalis, Thermus antranikianii, Thermus caldophilus, Thermus chliarophilus, Thermus flavus, Thermus igniterrae, Thermus lacteus, Thermus oshimai, Thermus ruber, Thermus rubens, Thermus scotoductus, Thermus silvanus, Thermus species Z05, Thermus species sps 17, Thermus thermophilus, Thermotoga maritima, Thermotoga neapolitana, Thermosipho africanus, Thermococcus litoralis, Thermococcus barossi, Thermococcus gorgonarius, Thermotoga maritima, Thermotoga neapolitana, Thermosiphoafricanus, Pyrococcus woesei, Pyrococcus horikoshii, Pyrococcus abyssi, Pyrodictium occultum, Aquifex pyrophilus and Aquifex aeolieus. In certain embodiment, the thermostable DNA polymerase is Taq polymerase.

Alternatively, the present invention may employ DNA polymerases having a 5' nuclease activity modified to have less polymerase activities.

According to an embodiment, the FEN (flap endonuclease) nuclease used is a 5' flap-specific nuclease.

The FEN nuclease suitable in the present invention comprises FEN nucleases obtained from a variety of bacterial species, including Sulfolobus solfataricus, Pyrobaculum aerophilum, Thermococcus litoralis, Archaeaglobus veneficus, Archaeaglobus profundus, Acidianus brierlyi, Acidianus ambivalens, Desulfurococcus amylolyticus, Desulfurococcus mobilis, Pyrodictium brockii, Thermococcus gorgonarius, Thermococcus zilligii, Methanopyrus kandleri, Methanococcus igneus, Pyrococcus horikoshii, Aeropyrum pernix, and Archaeaglobus veneficus.

Where the upstream primer is used in the step (a), the conditions for cleavage of the PTO may comprise extension reaction of the upstream primer.

According to an embodiment, the upstream primer is used in the step (a), a template-dependent polymerase is used for extension of the upstream primer and the template-dependent polymerase is identical to the enzyme having the 5' nuclease activity.

Optionally, the upstream primer is used in the step (a), a template-dependent polymerase is used for extension of the upstream primer and the template-dependent polymerase is different from the enzyme having the 5' nuclease activity.

### Step (c): Hybridization of the fragment released from the PTO with CTO

The fragment released from the PTO is hybridized with a CTO (Capturing and Templating Oligonucleotide).

The CTO comprises in a 3' to 5' direction (i) a capturing portion comprising a nucleotide sequence complementary to the 5'-tagging portion or a part of the 5'-tagging portion of the PTO and (ii) a templating portion comprising a nucleotide sequence non-complementary to the 5'-tagging portion and the 3-targeting portion of the PTO.

The CTO is acted as a template for extension of the fragment released from the PTO. The fragment serving as a primer is hybridized with the CTO and extended to form an extended duplex.

The templating portion may comprise any sequence so long as it is non-complementary to the 5'-tagging portion and the 3-targeting portion of the PTO. Furthermore, the templating portion may comprise any sequence so long as it can be acted as a template for extension of the fragment released from the PTO.

As described above, when the fragment having the 5'-tagging portion of the PTO is released, the capturing portion of the CTO may be designed to comprise a nucleotide sequence complementary to the 5'-tagging portion. When the fragment having the 5'-tagging portion and a 5'-end part of the 3'-targeting portion is released, the capturing portion of the CTO may be designed to comprise a nucleotide sequence complementary to the 5'-tagging portion and the 5'-end part of the 3'-targeting portion. When the fragment having a part of the 5'-tagging portion of the PTO is released, the capturing portion of the CTO may be designed to comprise a nucleotide sequence complementary to the part of the 5'-tagging portion.

Moreover, it is possible to design the capturing portion of the CTO with anticipating cleavage sites of the PTO. For example, where the capturing portion of the CTO is designed to comprise a nucleotide sequence complementary to the 5'-tagging portion, either the fragment having a part of the 5'-tagging portion or the fragment having the 5'-tagging portion can be hybridized with the capturing portion and then extended. Where the fragment comprising the 5'-tagging portion and a 5'-end part of the 3'-targeting portion is released, it may be hybridized with the capturing portion of the CTO designed to comprise a nucleotide sequence complementary to the 5'-tagging portion and then successfully extended although mismatch nucleotides are present at the 3'-end portion of the fragment. That is because primers can be extended depending on reaction conditions although its 3'-end contains some mismatch nucleotides (e.g. 1-3 mismatch nucleotides).

When the fragment comprising the 5'-tagging portion and a 5'-end part of the 3'-targeting portion is released, the 5'-end part of the capturing portion of the CTO (see Fig. 1) may be designed to have a nucleotide sequence complementary to the cleaved 5'-end part of the 3'-targeting portion, overcoming problems associated with mismatch nucleotides.

In an embodiment, the nucleotide sequence of the 5'-end part of the capturing portion of the CTO complementary to the cleaved 5'-end part of the 3'-targeting portion may be selected depending on anticipated cleavage sites on the 3'-targeting portion of the PTO. The nucleotide sequence of the 5'-end part of the capturing portion of the CTO complementary to the cleaved 5'-end part of the 3'-targeting portion may be 1-10 nucleotides, 1-5 nucleotides or 1-3 nucleotides in length.

The 3'-end of the CTO may comprise additional nucleotides not involved in hybridization with the fragment. Moreover, the capturing portion of the CTO may comprise a nucleotide sequence complementary only to a part of the fragment (e.g., a part of the fragment containing its 3'-end portion) so long as it is stably hybridized with the fragment.

The term used "capturing portion comprising a nucleotide sequence complementary to the 5'-tagging portion or a part of the 5'-tagging portion" is described herein to encompass various designs and compositions of the capturing portion of the CTO as discussed above.

The CTO may be designed to have a hairpin structure.

The length of the CTO may be widely varied. For example, the CTO is 6-1000 nucleotides, 6-500 nucleotides, 6-300 nucleotides, 6-100 nucleotides, 6-80 nucleotides, 6-60 nucleotides, 6-40 nucleotides, 15-1000 nucleotides, 15-500 nucleotides, 15-300 nucleotides, 15-100 nucleotides, 15-80 nucleotides, 15-60 nucleotides, 15-40 nucleotides, 20-1000 nucleotides, 20-500 nucleotides, 20-300 nucleotides, 20-100 nucleotides, 20-80 nucleotides, 20-60 nucleotides, 20-40 nucleotides, 30-1000 nucleotides, 30-500 nucleotides, 30-300 nucleotides, 30-100 nucleotides, 30-80 nucleotides, 30-60 nucleotides or 30-40 nucleotides in length. The capturing portion of the CTO may have any length so long as it is spedfically hybridized with the fragment released from the PTO. For example, the capturing portion of the CTO is 5-100 nucleotides, 5-60 nucleotides, 5-40 nucleotides, 5-30 nucleotides, 5-20 nucleotides, 10-100 nucleotides, 10-60 nucleotides, 10-40 nucleotides, 10-30 nucleotides, 10-20 nucleotides, 15-100 nucleotides, 15-60 nucleotides, 15-40 nucleotides, 15-30 nucleotides or 15-20 nucleotides in length. The templating portion of the CTO may have any length so long as it can act as a template in extension of the fragment released from the PTO. For example, the templating portion of the CTO is 1-900 nucleotides, 1-400 nucleotides, 1-300 nucleotides, 1-100 nucleotides, 1-80 nucleotides, 1-60 nucleotides, 1-40 nucleotides, 2-20 nucleotides, 2-900 nucleotides, 2-400 nucleotides, 2-300 nucleotides, 2-100 nucleotides, 2-80 nucleotides, 2-60 nucleotides, 2-40 nucleotides, 2-20 nucleotides, 5-900 nucleotides, 5-400 nucleotides, 5-300 nucleotides, 5-100 nucleotides, 5-80 nucleotides, 5-60 nucleotides, 5-40 nucleotides, 5-30 nucleotides, 10-900 nucleotides, 10-400 nucleotides, 10-300 nucleotides, 15-900 nucleotides, 15-100 nucleotides, 15-80 nucleotides, 15-60 nucleotides, 15-40 nucleotides or 15-20 nucleotides in length.

The 3'-end of the CTO may have a 3'-OH terminal. Specifically, the 3'-end of the CTO is blocked to prohibit its extension. The non-extendible blocking of the CTO may be achieved in accordance with conventional methods. For instance, the blocking may be performed by adding to the 3'-hydroxyl group of the last nucleotide of the CTO a chemical moiety such as biotin, labels, a phosphate group, alkyl group, non-nucleotide linker, phosphorothioate or alkane-diol. Alternatively, the blocking may be carried out by removing the 3'-hydroxyl group of the last nucleotide or using a nucleotide with no 3'-hydroxyl group such as dideoxynucleotide.

The fragment released from the PTO is hybridized with the CTO, providing a form suitable in extension of the fragment. Although an undigested PTO is also hybridized with the capturing portion of the CTO through its 5'-tagging portion, its 3'-targeting portion is not hybridized to the CTO which prohibits the formation of an extended duplex.

The hybridization in the step (c) can be described in detail with referring to descriptions in the step (a).

### Step (d): Extension of the fragment to form extended duplex

The extension reaction is carried out using the resultant of the step (c) and a template-dependent nucleic acid polymerase. The fragment hybridized with the capturing portion of the CTO is extended to form an extended strand containing an extended sequence complementary to the templating portion of the CTO, thereby forming the extended duplex. In contrast, uncleaved PTO hybridized with the capturing portion of the CTO is not extended such that no extended duplex is formed.

The extended strand comprises a label providing a detectable signal.

The term used herein "extended duplex" means a duplex formed by extension reaction in which the fragment hybridized with the capturing portion of the CTO is extended using the templating portion of the CTO as a template and the template-dependent nucleic acid polymerase.

The term used herein "extended strand" in conjunction with the fragment means a sequence composed of the fragment and its extended sequence. The term "extended sequence" in conjunction with the fragment refers to a newly synthesized sequence except for the fragment in the extended strand.

The template-dependent nucleic acid polymerase used in the step (d) may include any nucleic acid polymerases, for example, Klenow fragment of E. coli DNA polymerase I, a thermostable DNA polymerase and bacteriophage T7 DNA polymerase. Specifically, the polymerase is a thermostable DNA polymerase which may be obtained from a variety of bacterial species, including Thermus aquaticus (Taq), Thermus thermophilus (Tth), Thermus filiformis, Thermis flavus, Thermococcus literalis, Thermus antranikianii, Thermus caldophilus, Thermus chliarophilus, Thermus flavus, Thermus igniterrae, Thermus lacteus, Thermus oshimai, Thermus ruber, Thermus rubens, Thermus scotoductus, Thermus silvanus, Thermus species Z05, Thermus species sps 17, Thermus thermophilus, Thermotoga maritima, Thermotoga neapolitana, Thermosipho africanus, Thermococcus litoralis, Thermococcus barossi, Thermococcus gorgonarius, Thermotoga maritima, Thermotoga neapolitana, Thermosiphoafricanus, Pyrococcus furiosus(Pfu), Pyrococcus woesei, Pyrococcus horikoshii, Pyrococcus abyssi, Pyrodictium occultum, Aquifex pyrophilus and Aquifex aeolieus. More specifically, the template-dependent nucleic acid polymerase is Taq polymerase.

According to an embodiment, the enzyme having the 5' nuclease activity used in the step (b) is identical to the template-dependent nucleic acid polymerase used in the step (d). Specifically, the enzyme having the 5' nuclease activity used in the step (b), the template-dependent nucleic acid polymerase used for extension of the upstream primer and the template-dependent nucleic acid polymerase used in the step (d) are identical to one another.

The step (d) is performed under conditions such that the fragment hybridized with the capturing portion of the CTO is extended to produce the extended strand containing the extended sequence complementary to the templating portion of the CTO and the extended duplex is formed. Where the target nucleic acid sequence is absent, the PTO is not cleaved and the extended duplex therefore is not formed.

The extended strand comprises a label providing a detectable signal.

The label useful in this invention includes a wide variety of labels known to one of skill in the art.

According to an embodiment, the label contained in the extended strand is (i) a single label linked to the fragment released from the PTO, (ii) a label to be incorporated into the extended strand during the extension reaction, or (iii) a label originated from both the single label linked to the fragment and the label to be incorporated into the extended strand during the extension reaction.

The labeling systems used in this invention may be classified in terms of types of labels and labeled oligonucleotides as follows:

### (i) Single label linked to PTO fragment

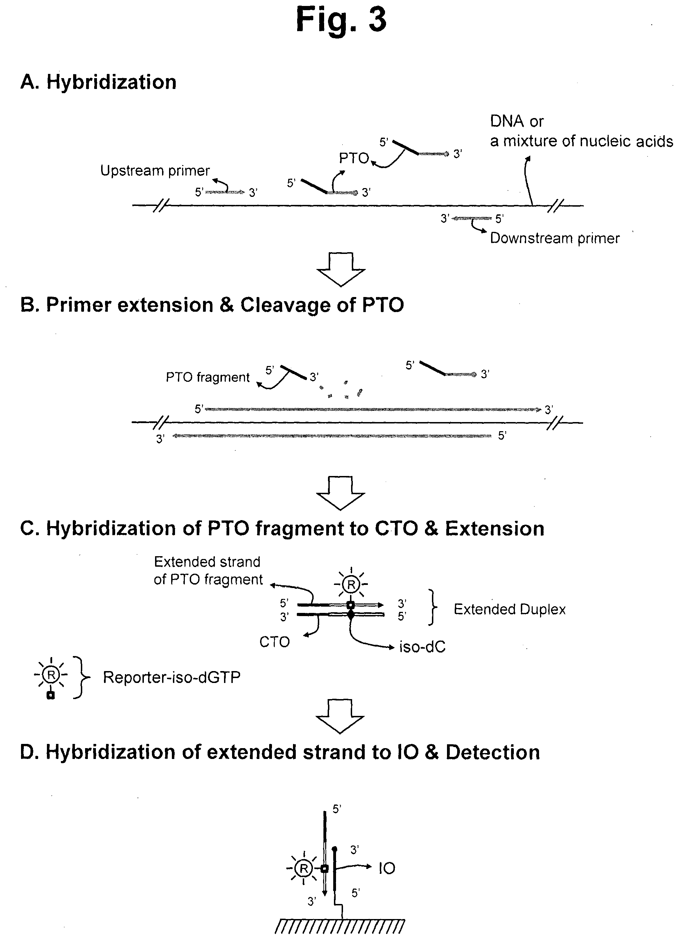

The present invention may provide signal for the presence of the extended strand indicating the presence of the target nucleic acid sequence by using the single label (see Figs. 2 and 3). The PTO fragment labeled with a single label is hybridized with the CTO and extended on the CTO to produce the extended strand, whereby the single label is contained in the extended strand. Afterwards, the extended strand is hybridized with the IO immobilized on a solid substrate to provide a detectable signal on the sold substrate, followed by detection of the signal indicative of the presence of the extended strand.

The single label includes a fluorescent label, a luminescent label, a chemiluminescent label, an electrochemical label and a metal label.

According to an embodiment, the single label includes a fluorescent label. The types of the fluorescent single label may be described with referring to the descriptions of interactive dual labels discussed hereinbelow.

The label may be linked to the PTO (e.g., the 5'-tagging portion of the PTO) by conventional methods. For instance, the label may be linked to the PTO through a spacer containing at least three carbon atoms (e.g., 3-carbon spacer, 6-carbon spacer or 12-carbon spacer). According to an embodiment, the single label on the PTO may be located at any site so long as the PTO fragment released from the PTO is ensured to contain the single label. According to an embodiment, the single label on the PTO may be located at any site of the 5'-tagging portion, for example, the 5'-end of the 5'-tagging portion.

### (ii) Label to be incorporated into the extended strand during the extension reaction

According to an embodiment, the label used in this invention is the label to be incorporated into the extended strand, the templating portion of the CTO comprises a nucleotide containing a first non-natural base, and the extension reaction of the step (d) is performed in the presence of a nucleotide containing a label and a second non-natural base with a specific binding affinity to the first non-natural, thereby incorporating the label into the extended strand.

The term used herein "non-natural base" refers to derivatives of natural bases such as adenine (A), guanine (G), thymine (T), cytosine (C) and uracil (U), which are capable of forming hydrogen-bonding base pairs. The term used herein "non-natural base" includes bases having different base pairing patterns (e.g., hydrogen bond patterns) from natural bases as mother compounds, as described, for example, in U.S. Pat. Nos. 5,432,272, 5,965,364, 6,001,983, and 6,037,120. The base pairing between non-natural bases involves two or three hydrogen bonds as natural bases. The base pairing between non-natural bases is also formed in a specific manner.

Specific examples of non-natural bases include the following bases in base pair combinations: iso-C/iso-G, iso-dC/iso-dG, K/X, H/J, and M/N (see U.S. Pat. No. 7,422,850).

The label to be incorporated into the extended strand is particularly liked to a nucleotide, more particularly to a nucleotide triphosphate. The label is particularly linked to a base.

The exemplified embodiment is described with reference to Fig. 3. The fragment released from the PTO hybridized with the target nucleic acid sequence is hybridized with the CTO containing iso-dC, iso-dG labeled with a fluorescent label is incorporated into an opposite site to iso-dC to introduce the fluorescent label into the extended strand during the extension reaction. Afterwards, the extended strand is hybridized with the IO immobilized on the solid substrate to provide a detectable signal on the solid substrate and the signal indicative of the presence of the extended strand is then detected.

According to an embodiment, the label used in this invention is the label to be incorporated into the extended strand and the extension reaction of the step (d) is performed in the presence of a nucleotide containing a single label, thereby incorporating the label into the extended strand. In such case, a plurality of the identical single label is incorporated into the extended strand in a dependent manner on the base type of the nucleotide containing the single label and the sequence of the templating portion of the CTO.

### (iii) Combination of the single label of the PTO fragment and the label to be incorporated into the extended strand

According to an embodiment, the label used in this invention is a combination of the single label linked to the fragment and the label to be incorporated into the extended strand during the extension reaction, and the single label and the incorporated label are an interactive dual label containing a pair of an acceptor and a donor.

For example, the PTO comprising the 5'-tagging portion labeled with a fluorescent single label (donor) is hybridized with the target nucleic acid sequence and then cleaved. The PTO fragment containing the fluorescent single label (donor) is hybridized with the CTO containing iso-dC and iso-dG labeled with an acceptor is incorporated into an opposite site to iso-dC during the extension reaction to introduce the acceptor into the extended strand, thereby forming an interactive dual label on the extended strand. Afterwards, the extended strand is hybridized with the IO immobilized on the solid substrate and irradiated using an excitation light for the donor to elicit energy transfer from the donor to the acceptor, thereby inducing signal change from the acceptor and providing a detectable signal on the solid substrate.

According to an embodiment, the pair of the acceptor and the donor is located at sites suitable in induction of quenching (or energy transfer) upon hybridizing the extended strand with the IO.

The acceptor and the donor molecules known to one of skill in the art are useful in the present invention.

Suitable fluorescent molecules and pairs of acceptor-donor (e.g., pairs of reporter-quencher) are disclosed in a variety of publications as follows: Pesce et al., editors, Fluorescence Spectroscopy (Marcel Dekker, New York, 1971); White et al., Fluorescence Analysis: A Practical Approach (Marcel Dekker, New York, 1970); Berlman, Handbook of Fluorescence Spectra of Aromatic Molecules, 2nd Edition (Academic Press, New York, 1971); Griffiths, Color AND Constitution of Organic Molecules (Academic Press, New York, 1976); Bishop, editor, Indicators (Pergamon Press, Oxford, 1972); Haugland, Handbook of Fluorescent Probes and Research Chemicals (Molecular Probes, Eugene, 1992); Pringsheim, Fluorescence and Phosphorescence (Interscience Publishers, New York, 1949); Haugland, R. P., Handbook of Fluorescent Probes and Research Chemicals, 6th Edition (Molecular Probes, Eugene, Oreg., 1996) U.S. Pat. Nos. 3,996,345 and 4,351,760.

The fluorescent labels as donor or acceptor molecules useful in the present invention may include any molecules known in the art. Examples of those are: Cy2^{™} (506), YO-PRO^{™}-1 (509), YOYO^{™}-1 (509), Calcein (517), FITC (518), FluorX^{™} (519), Alexa^{™} (520), Rhodamine 110 (520), Oregon Green^{™} 500 (522), Oregon Green^{™} 488 (524), RiboGreen^{™} (525), Rhodamine Green^{™} (527), Rhodamine 123 (529), Magnesium Green^{™}(531), Calcium Green^{™} (533), TO-PRO^{™}-1 (533), TOTO1 (533), JOE (548), BODIPY530/550 (550), Dil (565), BODIPY TMR (568), BODIPY558/568 (568), BODIPY564/570 (570), Cy3^{™} (570), Alexa^{™} 546 (570), TRITC (572), Magnesium Orange^{™} (575), Phycoerythrin R&B (575), Rhodamine Phalloidin (575), Calcium Orange^{™}(576), Pyronin Y (580), Rhodamine B (580), TAMRA (582), Rhodamine Red^{™} (590), Cy3.5^{™} (596), ROX (608), Calcium Crimson^{™} (615), Alexa^{™} 594 (615), Texas Red(615), Nile Red (628), YO-PRO^{™}-3 (631), YOYO^{™}-3 (631), R-phycocyanin (642), C-Phycocyanin (648), TO-PRO^{™}-3 (660), TOTO3 (660), DiD DilC(5) (665), Cy5^{™} (670), Thiadicarbocyanine (671), Cy5.5 (694), HEX (556), TET (536), Biosearch Blue (447), CAL Fluor Gold 540 (544), CAL Fluor Orange 560 (559), CAL Fluor Red 590 (591), CAL Fluor Red 610 (610), CAL Fluor Red 635 (637), FAM (520), Fluorescein (520), Fluorescein-C3 (520), Pulsar 650 (566), Quasar 570 (667), Quasar 670 (705) and Quasar 705 (610). The numeric in parenthesis is a maximum emission wavelength in nanometer.

### (iv) Intercalating dyes

According to an embodiment, the hybridization of the step (e) is performed in the presence of an intercalating dye, the label contained in the extended strand is an acceptor to receive signal from the intercalating dye, the signal from the intercalating dye intercalated into a hybrid between the extended strand and the IO is transferred to the acceptor, and the acceptor provides the detectable signal.

Exemplified intercalating dyes useful in this invention include SYBR^{™} Green I, PO-PRO^{™}-1, BO-PRO^{™}-1, SYTO^{™}43, SYTO^{™}44, SYTO^{™}45, SYTOX^{™}Blue , POPO^{™}-1, POPO^{™}-3, BOBO^{™}-1, BOBO^{™}-3, LO-PRO^{™}-1, JO-PRO^{™}-1, YO-PRO^{™}1, TO-PRO^{™}1, SYTO^{™}11, SYTO^{™}13, SYTO^{™}15, SYTO^{™}16, SYTO^{™}20, SYTO^{™}23, TOTO^{™}-3, YOYO^{™}3, GelStar^{™} and thiazole orange. The intercalating dyes intercalate specifically into double-stranded nucleic acid molecules to generate signals.

For example, the PTO comprising the 5'-tagging portion labeled with a fluorescent label (acceptor) is hybridized with the target nucleic acid sequence and then cleaved. The PTO fragment containing the fluorescent label (acceptor) is hybridized with the CTO and extended to form the extended strand. Afterwards, the extended strand is hybridized with the IO immobilized on the solid substrate in the presence of an intercalating dye as a donor to intercalate the intercalating dye into the hybrid. Following irradiation by using an excitation light for the donor, the energy transfer is elicited from the donor to the acceptor, thereby inducing signal change from the acceptor and providing a detectable signal on the solid substrate.

Alternatively, with no use of the label carried on in the extended strand, the hybridization of the step (e) may be performed in the presence of an intercalating dye and signal from the intercalating dye intercalated into a hybrid between the extended strand and the IO is detected to analyze the presence of the target nucleic acid sequence.

### Step (e): Hybridization between the extended strand with the IO

The extended strand in the step (d) is hybridized with an IO (immobilized oligonucleotide) immobilized on a solid substrate, thereby providing signal indicative of the presence of the target nucleic acid sequence on the solid substrate.

The IO is immobilized on the solid substrate and comprises a sequence complementary to the extended strand.

According to an embodiment, the IO comprises a sequence complementary to (i) all or a part of the extended sequence and (ii) a part of the fragment in the extended strand.

According to an embodiment, where the IO comprises the hybridizable sequence with the fragment in the extended strand, the step (e) is performed under conditions such that the hybridization solely between the hybridizable sequence of the IO and the fragment in the extended strand does not form a stable hybrid. Using the conditions contributes to inhibition of hybridization between the undigested PTO comprising a label and the IO, thereby inhibiting generation of non-target signal (false signal). The conditions include reaction temperatures, salt concentrations of reaction mixtures and pH of buffers.

In certain embodiment, the sequence of the IO is selected not to form a hybrid with an undigested PTO. For example, the complementarity between the IO and the 5'-tagging portion of an undigested PTO may be less than 90%, 70%, 50%, 30% or 20%, which may be varied depending on hybridization conditions for formation of the extended strand/IO hybrid.

According to an embodiment, the IO comprises a sequence complementary solely to the extended sequence.

The IO comprises no label.

The length of the IO may be widely varied. For example, the IO is 5-100 nucleotides, 5-80 nucleotides, 5-60 nucleotides, 5-40 nucleotides, 5-20 nucleotides, 5-10 nucleotides, 10-100 nucleotides, 10-80 nucleotides, 10-60 nucleotides, 10-40 nucleotides, 10-30 nucleotides, 10-20 nucleotides, 15-100 nucleotides, 15-80 nucleotides, 15-60 nucleotides, 15-40 nucleotides, 15-30 nucleotides, 15-20 nucleotides, 20-100 nucleotides, 20-80 nucleotides, 20-60 nucleotides, 20-40 nucleotides or 20-30 nucleotides in length.

In an embodiment of this invention, the IO is blocked at its 3'-end to prohibit its extension.

The IO is directly or indirectly immobilized through its 5'-end or 3'-end onto a solid substrate. In an embodiment of this invention, the IO is immobilized through its 5'-end or 3'-end onto a solid substrate. Specifically, the IO is immobilized through its 5'-end onto a solid substrate.

The IO may be immobilized on the surface of the solid substrate in a covalent or non-covalent manner. Where the immobilized IOs are immobilized indirectly onto the surface of the solid substrate, suitable linkers are used. The linkers useful in this invention may include any linkers utilized for probe immobilization on the surface of the solid substrate. For example, alkyl or aryl compounds with amine functionality, or alkyl or aryl compounds with thiol functionality serve as linkers for IO immobilization. In addition, poly (T) tail or poly (A) tail may serve as linkers. The poly (T) tail or poly (A) tail as linkers is not considered as a sequence of the IO.

According to an embodiment, the solid substrate used in the present nvention is a microarray. The microarray to provide a reaction environment in this invention may include any those known to one of skill in the art. All processes of the present invention, i.e., hybridization to target nucleic acid sequences, cleavage, extension, melting and fluorescence detection, are carried out on the microarray. The immobilized IOs on the microarray serve as hybridizable array elements. The solid substrate to fabricate microarray includes, but not limited to, metals (e.g., gold, alloy of gold and copper, aluminum), metal oxide, glass, ceramic, quartz, silicon, semiconductor, Si/SiO₂ wafer, germanium, gallium arsenide, carbon, carbon nanotube, polymers (e.g., polystyrene, polyethylene, polypropylene and polyacrylamide), sepharose, agarose and colloids. The solid substrate may be in the form of a dipstick, a plate, a particle (e.g., bead), an affinity column and a membrane. A plurality of immobilized IOs in this invention may be immobilized on an addressable region or two or more addressable regions on a solid substrate that may comprise 2-1,000,000 addressable regions. Immobilized IOs may be fabricated to produce array or arrays for a given application by conventional fabrication technologies such as photolithography, ink-jetting, mechanical microspotting, and derivatives thereof.

The present invention performed on the solid phase can detect simultaneously a plurality of target nucleic acid sequences even using a single type of a label because the labels on the extended strand hybridized with the immobilized IO are physically separated. In this regard, the number of target nucleic acid sequences to be detected by the present invention on the solid phase is not limited.

The CTO and the IO are competitive in hybridization with the extended strand and therefore the CTO is very likely to inhibit hybridization between the extended strand and the IO.

For instance, the formation of the extended duplex between the extended strand and the CTO may prohibit the hybridization between the extended strand and the IO.

According to an embodiment, the step (e) is performed under conditions favorable for hybridization of the extended strand through its single-stranded state with the IO, whereby inhibition of the hybridization between the extended strand and the IO by the formation of the extended duplex between the extended strand and the CTO is prevented.

According to an embodiment, the favorable conditions for hybridization of the extended strand through its single-stranded state with the IO over hybridization of the CTO with the IO are conditions permitting to increase the number of the extended strand in the single-stranded state in the step (e).

According to an embodiment, the conditions permitting to increase the number of the extended strand in the single-stranded state in the step (e) may be denaturation of the extended duplex prior to the hybridization between the extended strand and the IO. The denaturation allows to increase contact opportunity between the extended strand in the single-stranded state and the IO, thereby producing increasing- numbered hybrids between the extended strand and the IO. In certain embodiment, the present method further comprises the step of denaturing the extended duplex prior to the hybridization between the extended strand and the IO.

According to an embodiment, the conditions permitting to increase the number of the extended strand in the single-stranded state in the step (e) may be removal of the CTO prior to the hybridization between the extended strand and the IO. For instance, biotinlyated CTO may be removed through specific affinity to streptavidin or avidin. In an embodiment, the present method further comprises removing the CTO prior to the hybridization between the extended strand and the IO.

In certain embodiment, the removal of the CTO is carried out in a vessel different that for the hybridization between the extended strand and the IO.

Alternatively, the conditions permitting to increase the number of the extended strand in the single-stranded state in the step (e) are to use the PTO and the CTO in a mole ratio of the PTO to the CTO of more than 1 (particularly, the mole ratio of not less than 1.2, not less than 1.3, not less than 1.5, not less than 1.8, not less than 2, not less than 3, not less than 4, not less than 5, not less than 7 or not less than 10). By adjusting the mole ratio, the number of the extended strand remaining in the single-stranded state without forming the extended duplex with the CTO becomes increased.

The present inventors have intensive researches to enhance the detection efficiency of the present method and the following findings have been obtained: While the amount of the CTO is constantly present during the overall reactions of the present method, the extended strand is generated in a dependent manner on the presence of the target nucleic acid sequence. Upon increasing the number of the extended strand, the number of the extended strand in the single-stranded state with no hybridization with the CTO becomes increased. Therefore, the inhibition by the CTO against the hybridization between the extended strand and the IO may be excluded as much as possible, and the hybridization between the extended strand and the IO may fully occur to enhance the detection efficiency of the present method. Given PTO cleavage efficiency, hybridization efficiency between the PTO fragment and the CTO, and extension efficiency of the PTO fragment, it is advantageous to use the higher amount of the PTO than that of the CTO. Where the amount (i.e., mole ratio) of the PTO to the CTO is adjusted, the present method further comprises repeating all or some of the steps (a)-(f) with denaturation between repeating cycles. For instance, where the steps (a)-(d) are repeated with denaturation, the extended strand is repeatedly generated with maintaining the amount of the CTO. As such, the extended strand may be more efficiently generated, the extended strand in the single-stranded state may become more predominant, and furthermore the number of the extended strand may be larger than that of the CTO. In an embodiment, the repetition with denaturation may comprise a step to denature at least the extended duplex. Other components (e.g., upstream oligonucleotide, downstream primer and enzymes) may be used in amounts sufficient to not render to be limiting factors.

The term "denaturation" in the phrase "with denaturation between repeating cycles" or "repeated with denaturation" means to separate a double-stranded nucleic acid molecule to a single-stranded nucleic acid molecule.

In another approach for overcoming problems associated with hybridization competition between the CTO and the IO, the step (e) and/or (f) is performed under conditions enhancing a hybridization competitivity of the extended strand with the IO over that of the CTO with the extended strand.

The conditions permitting to enhance the hybridization competitivity of the extended strand with the IO over that of the CTO with the extended strand are to adjust Tₘ value of a hybrid between the extended strand and the IO and Tₘ value of a hybrid between the CTO and the extended strand. For example, the Tₘ value of the hybrid between the extended strand and the IO formed in the step (e) may be adjusted to be higher (e.g., 5°C higher, 10°C higher, 15°C higher or 20°C higher) than that of the hybrid between the CTO and the extended strand. The Tₘ value of the hybrid between the extended strand and the IO formed in the step (e) may be adjusted to have Tₘ value being different from that of the hybrid between the CTO and the extended strand as little as possible (e.g., 40°C lower, 35°C lower, 30°C lower, 25°C lower, 20°C lower, 15°C lower, 10°C lower, 5°C lower or identical Tₘ). The adjustment of Tₘ values may be performed by adjusting sequences or lengths of the CTO or the IO or using nucleotide analogues (e.g., PNA or LNA).

The sequences of the CTO and the IO can be selected irrespectively of a target nucleic acid sequence and their Tₘ values can be easily adjusted.

The term used herein "Tₘ" refers to a melting temperature at which half a population of double stranded nucleic acid molecules are dissociated to single-stranded molecules. The Tₘ value is determined by length and G/C content of nucleotides hybridized. The Tₘ value may be calculated by conventional methods such as Wallace rule (R.B. Wallace, et al., Nucleic Acids Research, 6:3543-3547(1979)) and nearest-neighbor method (SantaLucia J. Jr., et al., Biochemistry, 35:3555-3562(1996)); Sugimoto N., et al., Nucleic Acids Res., 24:4501-4505(1996)).

The IO may be designed to comprise a hybridizable sequence with the fragment in the extended strand, such that the hybrid between the IO and the extended strand may have relatively high Tₘ values compared with the case in that the IO is designed to comprise a hybridizable sequence only with the extended sequence.

The sequence design strategy afore-mentioned enables to employ CTOs in relatively short length compared with the case using only the extended sequence in the extended strand for hybridization between the extended strand and the IO. Using CTOs in relatively short length enables to apply the approach to adjust Tₘ values of the hybrids in more efficient manner. Particularly, where CTOs in relatively long length are used, the hybrid formed by the CTO has higher Tₘ values and the IO is compelled to have relatively long length or contain more nucleotide analogues for hybridization competition.

The sequence design strategy afore-mentioned enables to adjust Tₘ values in more convenient manner in the approach to adjust Tₘ values of the hybrids.

The hybrid forms between the extended strands and the CTOs seem to readily not access to the IOs integrated onto a solid substrate. Therefore, extended strands in the single-stranded state advantageously contact to the IOs integrated onto a solid substrate and they are hybridized with the IOs on a solid substrate. In certain embodiment, the extended strand in its single-stranded state is hybridized with the IO and exists in its single-stranded state.

### Step (f): Detection of signals

Finally, the detectable signal generated is detected on the solid substrate. The detectable signal is indicative of the presence of the extended strand which indicates the presence of the target nucleic acid sequence.

According to an embodiment, the signal indicative of the hybrid between the extended strand and the IO is provided at a predetermined temperature at which the extended strand and the IO form the hybrid and detected at the predetermined temperature.

According to an embodiment, the signal detection may be performed by detecting signals provided at end-point of the reaction.

According to an embodiment, the signal detection is performed in a real-time detection manner for detecting signal changes over reaction processing in a real-time manner.

Using confocal detection devices on a solid phase, signal existed only on the solid substrate may be detected with no influence of signal from labels present in a reaction solution.

According to an embodiment, the present invention is used for quantification of the target nucleic acid sequence.

According to an embodiment, the step (f) for detecting the signal is performed by detecting a signal on the solid substrate provided in a process of melting a hybrid between the extended strand and the IO over a range of temperatures or melting and hybridizing the hybrid. Such signal detection may be carried out by melting analysis known to one of skill in the art.

The melting analysis may be carried out by various processes known to one of skill in the art. The term used herein "melting analysis" is used to intend to encompass a melting analysis and a hybridization analysis in a narrow meaning. THe melting analysis includes a method to measure signals at two different temperatures, melting (hybridization) curve analysis, melting (hybridization) pattern analysis and melting (hybridization) peak analysis.

The melting curve or hybridization curve may be obtained by conventional technologies, for example, as described in U.S. Pat Nos. 6,174,670 and 5,789,167, Drobyshev et al, Gene 188: 45(1997); Kochinsky and Mirzabekov Human Mutation 19:343(2002); Livehits et al J. Biomol. Structure Dynam. 11:783(1994); and Howell et al Nature Biotechnology 17:87(1999). For example, a melting curve or hybridization curve may consist of a graphic plot or display of the variation of the output signal with the parameter of hybridization stringency. Output signal may be plotted directly against the hybridization parameter. Typically, a melting curve or hybridization curve will have the output signal, for example fluorescence, which indicates the degree of duplex structure (i.e. the extent of hybridization), plotted on the Y-axis and the hybridization parameter on the X axis.

The melting (hybridization) curve analysis and melting (hybridization) peak analysis are described in more detail with referring to descriptions of US Pat No 8,039,21.

In certain embodiment, the melting analysis is performed at least twice for quantitative analysis. The area or height of melting peaks obtained in melting analysis is affected by the amount of the extended strand, providing information as to the initial amount of target nucleic acid sequences.

In certain embodiment, the present invention comprises the steps of: (i) repeating the steps (a)-(d) with denaturation between the repeating cycles to increase the number of the extended strand; (ii) performing a melting analysis for the hybrid between the IO and the extended strand; and (iii) repeating at least twice the steps (i) and (ii). The data may be obtained in a predetermined repetition interval with at least two points. Then, the cycle number of melting analysis at which the melting peak area or height crosses a threshold value is measured to quantify the amount of target nucleic acid sequences. Alternatively, melting analysis results (e.g., the melting peak area or height) are plotted against each cycle number (or cumulative cycle number) of melting analysis and compared, thereby quantifying the amount of target nucleic acid sequences.

In certain embodiment, the present method further comprises the step of washing the solid substrate before detection of signals.

The primer, PTO, CTO and IO may be comprised of naturally occurring dNMPs and/or NMPs. Alternatively, the primer, PTO, CTO and IO may be comprised of modified nucleotide or non-natural nucleotide such as PNA (peptide nucleic acid, see PCT Publication No. WO 92/20702) and LNA (locked nucleic acid, see PCT Publication Nos. WO 98/22489, WO 98/39352 and WO 99/14226). The primer, PTO, CTO and IO may comprise universal bases such as deoxyinosine, inosine, 1-(2'-deoxy-beta-D-ribofuranosyl)-3-nitropyrrole and 5-nitroindole. The term "universal base" refers to one capable of forming base pairs with each of the natural DNA/RNA bases with little discrimination between them.

As described above, the PTO may be cleaved at a site located in a 3'-direction apart from the 3'-end of the 5'-tagging portion of the PTO. The cleavage site may be located at the 5'-end part of the 3'-targeting portion of the PTO. Where the PTO fragment comprises the 5'-end part of the 3'-targeting portion of the PTO, a site of the CTO hybridized with the 5'-end part of the 3'-targeting portion may comprise a universal base, degenerate sequence or their combination. For instance, if the PTO is cleaved at a site located one nucleotide in a 3'-direction apart from the 3'-end of the 5'-tagging portion of the PTO, it is advantageous that the 5'-end part of the capturing portion of the CTO comprises a universal base for hybridization with the nucleotide. If the PTO is cleaved at a site located two nucleotides in a 3'-direction apart from the 3'-end of the 5'-tagging portion of the PTO, it is advantageous that the 5'-end of the capturing portion of the CTO comprises a degenerate sequence and its 3'-direction-adjacent nucleotide comprises a universal base. As such, where the cleavage of the PTO occurs at various sites of the 5'-end part of the 3'-targeting portion, the utilization of universal bases and degenerate sequences in the CTO is useful. In addition, where the PTOs having the same 5'-tagging portion are used for screening multiple target nucleic acid sequences under upstream primer extension-dependent cleavage induction, the PTO fragments having different 5'-end parts of the 3'-targeting portion may be generated. In such cases, universal bases and degenerate sequences are usefully employed in the CTO. The strategies using universal bases and degenerate sequences in the CTO ensure to use one type or minimal types of the CTO for screening multiple target nucleic acid sequences.

According to an embodiment, the present method further comprises repeating all or some of the steps (a)-(f) with denaturation between repeating cycles. This repetition permits to amplify the target nucleic acid sequence and/or the target signal.

In certain embodiment, the amount of the PTO is excess and the cycle number of the repetition is adjusted to produce the extend strand in excessive amount.

According to an embodiment, the steps (a)-(f) are performed in a reaction vessel or in separate reaction vessels. For example, the steps (a)-(b), (c)-(d) and (e)-(f) are performed in different reaction vessels from one another.

In certain embodiment, the steps (a)-(b) and (c)-(f) are performed in a reaction vessel or in separate reaction vessels. The steps (a)-(b) and (c)-(f) may be carried out even in a reaction vessel in differential manner from each other. For example, where the hybridization between the 3'-targeting portion of the PTO and the target nucleic acid sequence may occur under higher stringent conditions the hybridization between the fragment and the CTO or between the extended strand and the IO, the repetition of the steps (a)-(b) may undertake with not accompanied by processing of the steps (c)-(f) and the steps (c)-(f) may undertake after completion of the steps (a)-(b).

In certain embodiment, the steps (a)-(d) and (e)-(f) are performed in two different reaction vessels from each other.

In certain embodiment, the steps (a)-(b) are repeatedly carried out with denaturation between repeating cycles.

It would be obvious to one of skill in the art that the repetition of some steps, the intervention of the denaturation into the repetition, the separation of some step(s) and the detection time may be widely varied within the spirit of the invention.

In certain embodiment, where the repetition with denaturation between repeating cycles is carried out by using a reaction mixture of the step (a) containing an upstream primer, it is performed in the presence of a downstream primer, e.g., by PCR method.

In certain embodiment, where the repetition with denaturation between repeating cycles is carried out by using a reaction mixture of the step (a) containing an upstream probe, it is performed in the presence of a downstream primer.

The term used herein "nucleic acid sample" refers to a non-biological sample (e.g., food, water, air, soil and waste) or biological sample containing nucleic acid molecules. The biological sample may be derived from animal, plant, human, fungus, bacterium and virus. The biological sample may be cell, tissue, or fluid from a biological source, blood, plasma, serum, serum, plasma, lymph, milk, urine, faeces, ocular fluid, saliva, semen, brain extracts, spinal cord fluid, appendix, spleen and tonsillar tissue extracts.

The present invention does not require that target nucleic acid sequences to be detected and/or amplified have any particular sequence or length, including any DNA (gDNA and cDNA) and RNA molecules. The target nucleic acid sequence may be in a single- or double-strand.

Where a mRNA is employed as starting material, a reverse transcription step is necessary prior to performing annealing step, details of which are found in Joseph Sambrook, et al., Molecular Cloning, A Laboratory Manual, Cold Spring Harbor Laboratory Press, Cold Spring Harbor, N.Y.(2001); and Noonan, K. F. et al., Nucleic Acids Res. 16:10366 (1988). For reverse transcription, a random hexamer or an oligonucleotide dT primer hybridizable to mRNA can be used.

The target nucleic acid sequence includes any naturally occurring prokaryotic, eukaryotic (for example, protozoans and parasites, fungi, yeast, higher plants, lower and higher animals, including mammals and humans), viral (for example, Herpes viruses, HIV, influenza virus, Epstein-Barr virus, hepatitis virus, polio virus, etc.), or viroid nucleic acid. The nucleic acid molecule can also be any nucleic acid molecule which has been or can be recombinantly produced or chemically synthesized. Thus, the nucleic acid sequence may or may not be found in nature. The target nucleic acid sequence may include known or unknown sequences.

The present invention is also useful in detection of a nucleotide variation. In certain embodiment, the target nucleic acid sequence comprises a nucleotide variation. The term "nucleotide variation" used herein refers to any single or multiple nucleotide substitutions, deletions or insertions in a DNA sequence at a particular location among contiguous DNA segments that are otherwise similar in sequence. Such contiguousDNA segments include a gene or any other portion of a chromosome. These nucleotide variations may be mutant or polymorphic allele variations. For example, the nucleotide variation detected in the present invention includes SNP (single nucleotide polymorphism), mutation, deletion, insertion, substitution and translocation. Exemplified nucleotide variation includes numerous variations in a human genome (e.g., variations in the MTHFR (methylenetetrahydrofolate reductase) gene), variations involved in drug resistance of pathogens and tumorigenesis-causing variations. The term nucleotide variation used herein includes any variation at a particular location in a nucleic acid sequence. In other words, the term nucleotide variation includes a wild type and its any mutant type at a particular location in a nucleic acid sequence.

In the present invention for detection of a nucleotide variation in a target nucleic acid sequence, where primers or probes used have a complementary sequence to the nucleotide variation in the target nucleic acid sequence, the target nucleic acid sequence containing the nucleotide variation is described herein as a matching template. Where primers or probes used have a non-complementary sequence to the nucleotide variation in the target nucleic acid sequence, the target nucleic acid sequence containing the nucleotide variation is described herein as a mismatching template.

For detection of nucleotide variations, the 3'-end of the upstream primer may be designed to be opposite to a site of a nucleotide variation in a target nucleic acid sequence. According to an embodiment, the 3'-end of the upstream primer has a complementary sequence to the nucleotide variation in a target nucleic acid sequence. The 3'-end of the upstream primer having a complementary sequence to the nucleotide variation in the target nucleic acid sequence is annealed to the matching template and extended to induce cleavage of the PTO. The resultant PTO fragment is hybridized with the CTO, extended and hybridized with the IO to provide the target signal. In contrast, where the 3'-end of the upstream primer is mismatched to a nucleotide variation in a mismatching template, it is not extended under conditions that annealing of the 3'-end of primers is essential for extension even when the upstream primer is hybridized with the mismatching template, thereby resulting in no generation of the target signal.

Alternatively, it is possible to use PTO cleavage depending on the hybridization of PTO having a complementary sequence to a nucleotide variation in a target nucleic acid sequence. For example, under controlled conditions, a PTO having a complementary sequence to the nucleotide variation in the target nucleic acid sequence is hybridized with the matching template and then cleaved. The resultant PTO fragment is hybridized with the CTO, extended and hybridized with the IO to provide the target signal. While, under the controlled conditions, the PTO is not hybridized with a mismatching template having non-complementary sequence in the nucleotide variation position and not cleaved. Particularly, in this case, the complementary sequence to the nucleotide variation in the PTO is positioned at its middle of the 3'-targeting portion of the PTO.

According to an embodiment, the use of an artificial mismatch nucleotide enhances discrimination potential of the PTO to nucleotide variations.

Alternatively, the present invention uses the PTO having the nucleotide variation discrimination site positioned on the 5'-end part of the 3'-targeting portion for selectivity of the PTO to a specific nucleotide variation. The 5'-end part of the 3'-targeting portion of the PTO is positioned to a nucleotide variation in a target nucleic acid sequence for the detection of the nucleotide variation and the 5'-end part of the 3'-targeting portion of the PTO has a complementary sequence to the nucleotide variation in a target nucleic acid sequence.

Where the PTO is hybridized with the target nucleic acid sequence (i.e., match template) having the nucleotide variation complementary to the nucleotide variation discrimination site, the 5'-end part of the 3'-targeting portion forms a double strand with the match template; however, where the PTO is hybridized with a target nucleic acid sequence (i.e., mismatch template) having a nucleotide variation non-complementary to the nucleotide variation discrimination site, the 5'-end part of the 3'-targeting portion does not form a double strand with the mismatch template.

The term used herein "nucleotide variation discrimination site" with reference to the PTO is a complementary sequence on the 5'-end part of the 3'-targeting portion of the PTO to a nucleotide variation in a target nucleic acid sequence.

According to an embodiment, the nucleotide variation discrimination site is located within 10 nucleotides, more preferably 8 nucleotides, still more preferably 6 nucleotides, still much more preferably 4 nucleotides, 3 nucleotides, 2 nucleotides or 1 nucleotide apart from the 5'-end of the 3'-targeting portion of the PTO. Preferably, the nucleotide variation discrimination site is located at the 5'-end of the 3'-targeting portion of the PTO.

The term "site" with reference to either nucleotide variation discrimination site of probes or nucleotide variation site on target sequences is used herein to encompass not only a single nucleotide but also a plurality of nucleotides.

It is noteworthy that such distinct hybridization patterns on the nucleotide variation of interest are responsible for differences in initial cleavage sites of the PTO, thereby producing two types of PTO fragments to give signal differentiation depending on the presence of the nucleotide variation of interest.

In the presence of the nucleotide variation of interest, a first fragment is generated by cleavage of hybrid between the PTO and matching template, and in the absence of the nucleotide variation of interest, a second fragment is generate by cleavage of hybrid between the PTO and mismatching template. The second fragment comprises an additional 3'-end portion rendering the second fragment to be different from the first fragment.

In an embodiment for the detection of a single nucleotide variation, the 5'-end of the 3'-targeting portion of the PTO has a complementary sequence to the single nucleotide variation in a target nucleic acid sequence. As described above, the cleavage of the PTO hybridized with a matching template may be induced at a site immediately adjacent in a 3'-direction to the 5'-end of the 3'-targeting portion of the PTO, for example, under upstream primer extension-dependent cleavage induction. The 3'-end of the PTO fragment has the complementary nucleotide to the single nucleotide variation. The PTO fragment is hybridized with a CTO having a capturing portion comprising a sequence corresponding to the nucleotide variation and then extended to form the extended strand. The extended strand is hybridized with the IO to provide the target signal. If the same PTO is hybridized with a mismatching template having the identical sequence to the matching template except for the single nucleotide variation, the cleavage of the PTO may occur at a site two nucleotides apart in a 3'-direction from the 5'-end of the 3'-targeting portion of the PTO. The 3'-end of the PTO fragment has the further cleaved nucleotide than the complementary nucleotide to the single nucleotide variation. Where the site of the CTO hybridized with the additional-cleaved nucleotide is designed to have a non-complementary sequence to the further cleaved nucleotide, the 3'-end of the PTO fragment is not hybridized with the CTO, resulting in no extension of the PTO fragment in a controlled condition.

According to an embodiment, a cleavage site of the PTO having a complementary sequence to the nucleotide variation at its 5'-end part of the 3'-targeting portion is different depending on hybridization with a matching template or with a mismatching template, such that the PTO fragment released from either hybridization event has different sequence preferably, in its 3'-end part, more preferably, in its 3'-end.

According to an embodiment, the selection of the nucleotide sequence of CTO in consideration of the difference in 3'-end parts of the PTO fragments allows to discriminate the matching template from the mismatching template.

According to an embodiment, the production of either the PTO fragments may be distinctly detected by an extension reaction on the CTO.

According to an embodiment, the CTO has a sequence selected such that the CTO is not hybridized with the additional 3'-end portion of the second fragment to prevent the second fragment from extension when the second fragment is hybridized with the capturing portion of the CTO.

As described above, the extension of the first fragment is detected by hybridization with the IO.

According to an embodiment, the 5'-end part of the 3'-targeting portion of the PTO comprises a non-base pairing moiety located within 1-10 nucleotides (more preferably 1-5 nucleotides) apart from the nucleotide variation discrimination site.

The non-base pairing moiety prevents the 5'-end part of the 3'-targeting portion from formation of a double strand with the target nucleotide sequence when the PTO is hybridized with the target nucleic acid sequence having the nucleotide variation non-complementary to the variation discrimination site. The use of the non-base pairing moiety (e.g., artificial mismatch nucleotide) enhances discrimination potential of the PTO to nucleotide variations.

According to an embodiment, the non-base pairing moiety does not inhibit the formation of a double strand between the 5'-end part and the target nucleic acid sequence when the PTO is hybridized with the target nucleic acid sequence having the nucleotide variation complementary to the nucleotide variation discrimination site.

According to an embodiment, the non-base pairing moiety widens the distance between the initial cleavage site on the hybrid of the PTO and the matching template and the initial cleavage site on the hybrid of the PTO and the mismatching template.

According to an embodiment, the introduction of a non-base paring moiety sequence enables the initial cleavage site to be adjusted, particularly the initial cleavage site on the hybrid of the PTO and the mismatching template.

According to an embodiment, the non-base pairing moiety is located downstream of the nucleotide variation discrimination site.

The non-base pairing moiety includes any moieties not forming a base pair between target nucleic acid sequences. Preferably, the non-base pairing moiety is (i) a nucleotide comprising an artificial mismatch base, a natural/non-natural base incapable of base-pairing, a base modified to be incapable of base pairing or a universal base, (ii) a non-base pairing nucleotide modified to be incapable of base pairing, or (iii) a non-base pairing chemical compound.

For example, the non-base pairing moiety includes alkylene group, ribofuranosyl naphthalene, deoxy ribofuranosyl naphthalene, metaphosphate, phosphorothioate linkage, alkyl phosphotriester linkage, aryl phosphotriester linkage, alkyl phosphonate linkage, aryl phosphonate linkage, hydrogen phosphonate linkage, alkyl phosphoroamidate linkage and aryl phosphoroamidate linkage. Conventional carbon spacers are also used as non-base pairing moieties. Universal bases as non-base pairing moieties are useful in adjusting cleavage sites of the PTO.

As base pairs containing universal bases such as deoxyinosine, 1-(2'-deoxy-beta-D-ribofuranosyl)-3-nitropyrrole and 5-nitroindole have a lower binding strength than those between natural bases, universal bases may be employed as non-base pairing moieties under certain hybridization conditions.

The non-base pairing moiety introduced into the 5'-end part has preferably 1-10, more preferably 1-5, still more preferably 1-2 moieties. A plurality of non-base pairing moieties in the 5'-end part may be present in a consecutive or intermittent manner. In certain embodiment, the non-base pairing moiety has 2-5 consecutive moieties.

In certain embodiment, the non-base pairing moiety is a non-base pairing chemical compound.

According to an embodiment, the nucleotide variation discrimination site and the non-base pairing moiety of the PTO are located within 10 nucleotides (more preferably 8 nucleotides, 7 nucleotides, 6 nucleotides, 5 nucleotides, 4 nucleotides, 3 nucleotides, 2 nucleotides or 1 nucleotide, still more preferably 1 nucleotide) apart from the 5'-end of the 3'-targeting portion.

Where a probe comprising both at its 5'-end a non-complementary tail to a target nucleic acid sequence and at its 5'-end portion a nucleotide variation discrimination site is hybridized with a mismatch template to a nucleotide at the nucleotide variation discrimination site, the 5'-end portion may form a single strand and the other portions may form a double strand with the target nucleic acid sequence While such a probe is different from the PTO in terms of probe design, it may be considered as probes having the PTO structure upon hybridization with mismatching templates. Therefore, signal may be generated by the present analysis method using the PTO. The application may be useful in detection of target nucleic acid sequences having a nucleotide variation non-complementary to the nucleotide variation discrimination site of probes.

The PCE-IH assay in conjunction with preferential amplification methods using an amplification blocker may effectively detect nucleotide variations in minority.

Clinical samples usually contain the wild-type allele in abundance and the mutant allele in minority. In an amplification step, the amplification of the abundant wild-type allele is likely to consume most reagents for amplification and therefore the mutant allele is not amplified to prohibit signal generation. For overcoming the shortcomings, there have been suggestions to selectively amplify the mutant allele with preventing amplification of the wild-type allele.

Representatively, methods using oligonucleotides containing PNA or LNA as an amplification blocker have been reported (US 2004/0014105, US 7,803,543, US 8,206,929, H. Orum., Nucleic Acids Research 21:5332-5336(1993) A. Senescau et al., Journal of Clinical Microbiology, 3304-3308(2005), Y. Nagai et al., Cancer Res 65:7276-7282(2005), Henrik et al., Nucleic Acid Research 21:5332-5336(1993) and Luo et al., Nucleic Acid Research Vol. 34, No 2 e12 (2006)). These methods have been called as "clamping method".

In general, the amplification blockers for clamping are hybridized only with templates having perfectly complementary sequence to the amplification blockers under the same condition, which are designed not to be hybridized with templates having even single mismatch. The template hybridized with the amplification blocker inhibiting primer annealing or chain elongation is not amplified and only that not hybridized with the amplification blocker is amplified. Nucleic acid analogues such as PNA and LNA are useful as amplification blockers in the senses that they show significant Tₘ differences for even a single base difference.

Where polymerases used have nuclease activity, the amplification blocker is required to possess resistant to nucleases activity.

Where two variant types which have different sequences from each other in a nucleotide variation region of a target nucleic acid sequence are present in a sample, the amplification blocker permits to amplify a target nucleic acid sequence having the nucleotide variation of interest and not to amplify a target nucleic acid sequence having the other nucleotide variation, enabling to detect the nucleotide variation of interest in more efficient manner. In particular, the utilization of amplification blocker is very useful in detection of the mutant allele in minority from clinical samples containing the wild-type allele in abundance and the mutant allele in minority.

Although the amplification blocker is used, the amplification of the wild-type allele may be not completely prevented. However, the PCE-IH assay using the PTO with excellent discrimination potential to nucleotide variations allows to detect mutant alleles in minority that are hardly detected by conventional technologies.

In certain embodiment, the amplification blocker located downstream of a primer blocks extension of the primer.

In certain embodiment, the amplification blocker is an oligonucleotide prepared with natural nucleoside/nucleotide, nucleoside/nucleotide analogue or their combination.

In certain embodiment, the amplification blocker comprises nucleosides/nucleotides having a backbone resistant to 5' nuclease activity. The nucleosides/nucleotides with a backbone resistant to the 5 nuclease activity include any one known to one of skill in the art. For example, it includes various phosphorothioate linkages, phosphonate linkages, phosphoroamidate linkages and 2'-carbohydrates modifications. In certain embodiment, nucleotides having a backbone resistant to the 5 nuclease include phosphorothioate linkage, alkyl phosphotriester linkage, aryl phosphotriester linkage, alkyl phosphonate linkage, aryl phosphonate linkage, hydrogen phosphonate linkage, alkyl phosphoroamidate linkage, aryl phosphoroamidate linkage, phosphoroselenate linkage, 2'-O-aminopropyl modification, 2'-O-alkyl modification, 2'-O-allyl modification, 2'-O-butyl modification, -anomeric oligodeoxynucleotide and 1-(4'-thio- -D-ribofuranosyl) modification.

According to an embodiment, the amplification blocker comprises peptide nucleic acid (PNA), locked nucleic acid (LNA), Morpholino, glycol nucleic acid (GNA), threose nucleic acid (TNA), bridged nucleic acids (BNA), N3' -P5' phosphoramidate (NP) oligomers, minor groove binder-linked-oligonucleotides (MGB-linked oligonucleotides), phosphorothioate (PS) oligomers, C₁-C₄alkylphosphonate oligomers, phosphoramidates, β-phosphodiester oligonucleotides, a-phosphodiester oligonucleotides or combination thereof.

In certain embodiment, the amplification blocker has resistance to 5' nuclease activity and its Tₘ value in hybridization is greatly affected by even by a single mismatch. Representative examples having the afore-mentioned properties are amplification blockers containing PNA or LNA.

The amplification blocker may have any length. For instance, the amplification blocker is 5-100 nucleotides, 5-80 nucleotides, 5-50 nucleotides, 5-40 nucleotides, 5-30 nucleotides, 10-100 nucleotides, 10-80 nucleotides, 10-50 nucleotides, 10-40 nucleotides, 10-30 nucleotides, 15-100 nucleotides, 15-80 nucleotides, 15-50 nucleotides, 15-40 nucleotides, 15-30 nucleotides, 20-100 nucleotides, 20-80 nucleotides, 20-50 nucleotides, 20-40 nucleotides or 20-30 nucleotides in length.

In certain embodiment, the amplification blocker is blocked at its 3'-end to prohibit its extension.

The nucleotide variation discrimination site (i.e., a complementary region to the non-target nucleotide variation) of the amplification blocker to be opposite to the nucleotide variation region on the target nucleic acid sequence may be located on any site of the amplification blocker, so long as it inhibits the amplification of the target nucleic acid sequence having the non-target nucleotide variation but does not inhibit the amplification of the target nucleic acid sequence having the target nucleotide variation.

In certain embodiment, the nucleotide variation discrimination site of the amplification blocker may be located at its 5'-end portion, middle portion or 3'-end portion.

The amplification blocker may be more than 200 nucleotides, more than 100 nucleotides, more than 50 nucleotides, more than 30 nucleotides, more than 20 nucleotides, more than 10 nucleotides, more than 5 nucleotides, more than 2 nucleotides, or more than 1 nucleotide apart from a primer that is located upstream of the amplification blocker. The amplification blocker may be immediately adjacent to the primer.

Where the PCE-IH assay in conjunction with the amplification blocker is employed to detect nucleotide variation, the PTO and the amplification blocker may be designed to be located on the same strand or different strands of the double strand of a target nucleic acid sequence. Where designed to be located on the same strand, the PTO and the amplification blocker may be interactively affected in hybridization with a target nucleic acid sequence and adjustment of reaction conditions or sequences may be required.

According to an embodiment, the target nucleic acid sequence used in the present invention is a pre-amplified nucleic acid sequence by an amplification primer.

The advantages of the present invention may be highlighted in the simultaneous (multiplex) detection of at least two target nucleic acid sequences.

According to an embodiment, the method is performed to detect at least two types of target nucleic acid sequences; wherein the upstream oligonucleotide comprises at least two types of oligonucleotides, the PTO comprises at least two types of the PTOs, the CTO comprises at least two types of the CTO, and the IO comprises at least two types of the IO.

According to an embodiment, the present invention is performed using at least two types of downstream primers to the PTO.

The target nucleic acid sequence to be detected by the present invention includes a wide variety of nucleic acid sequences, e.g., sequences in a genome, artificially isolated or fragmented sequences and synthesized sequences (e.g., cDNA sequences and barcode sequences). For instance, the target nucleic acid sequence includes nucleic acid marker sequences for Immuno-PCR (IPCR). IPCR employs conjugates between nucleic acid marker sequences and antibodies together with PCR, which is widely applied for detecting various types of targets including proteins (see Sano et al., Science 258 pp: 120-122(1992), U.S. Pat. No. 5,665,539, Niemeyer et al., Trends in Biotechnology 23 pp:208-216(2005), U.S. Pat. Pub. No. 2005/0239108 and Ye et al., Journal of Environmental Science 22 pp:796-800(2010)). The target nucleic acid molecule of the present invention includes nucleic acid markers as used in IPCR method and the present invention may be applied to detect nucleic acid markers in IPCR method.

### An Embodiment with Amplification of a Target Nucleic Acid Sequence

The present invention may be carried out simultaneously with amplification of a target nucleic acid sequence using a primer pair composed of an upstream primer and a downstream primer capable of synthesizing the target nucleic acid sequence.

### PCE-IH assay based on upstream oligonucleotide-independent 5' nuclease activity

In the first aspect of the present invention, there is provided a method for detecting a target nucleic acid sequence in a nucleic acid sample by a PCE-IH (PTO Cleavage and Extension-Dependent Immobilized Oligonucleotide Hybridization) assay on a solid phase, comprising:
(a) hybridizing the target nucleic acid sequence with a PTO (Probing and Tagging Oligonucleotide); wherein the PTO comprises (i) a 3'-targeting portion comprising a hybridizing nucleotide sequence complementary to the target nucleic acid sequence and (ii) a 5'-tagging portion comprising a nucleotide sequence non-complementary to the target nucleic acid sequence; wherein the 3'-targeting portion is hybridized with the target nucleic acid sequence and the 5'-tagging portion is not hybridized with the target nucleic acid sequence;
(b) contacting the resultant of the step (a) to the enzyme having a 5' nuclease activity under conditions for cleavage of the PTO; wherein the PTO is cleaved by the enzyme having the 5' nuclease activity such that the cleavage releases a fragment comprising the 5'-tagging portion or a part of the 5'-tagging portion of the PTO;
(c) hybridizing the fragment released from the PTO with a CTO (Capturing and Templating Oligonucleotide); wherein the CTO comprises in a 3' to 5' direction (i) a capturing portion comprising a nucleotide sequence complementary to the 5'-tagging portion or a part of the 5'-tagging portion of the PTO and (ii) a templating portion comprising a nucleotide sequence non-complementary to the 5'-tagging portion and the 3'-targeting portion of the PTO; wherein the fragment released from the PTO is hybridized with the capturing portion of the CTO;
(d) performing an extension reaction using the resultant of the step (c) and a template-dependent nucleic acid polymerase; wherein the fragment hybridized with the capturing portion of the CTO is extended to produce an extended strand containing an extended sequence complementary to the templating portion of the CTO and an extended duplex is formed; wherein the extended strand comprises a label;
(e) hybridizing the extended strand with an IO (immobilized oligonucleotide) immobilized on a solid substrate; wherein the IO comprises a sequence complementary to the extended strand; wherein the hybridization between the extended strand with the IO provides a detectable signal on the solid substrate; wherein the sequence of the IO is selected not to form a hybrid with an undigested PTO and
(f) detecting the signal indicative of the presence of the extended strand; the presence of the extended strand indicates the presence of the target nucleic acid sequence.

Interestingly, the present method based on upstream oligonucleotide-independent 5' nuclease activity practically provides target signals by the PCE-IH assay even no use of upstream oligonucleotides.

For the present method, conventional enzymes having upstream oligonucleotide-independent 5' nuclease activity may be used. Among template-dependent polymerases having 5' nuclease activity, there are several enzymes having upstream oligonucleotide-independent 5' nuclease activity, e.g., Taq DNA polymerase.

Considering amplification of target nucleic acid sequences and cleavage efficiency of the PTO, the PCE-IH assay of the present invention is preferably performed using upstream oligonucleotides.

According to an embodiment, the method further comprises repeating at least twice all or some (e.g., steps (a)-(b), (a)-(d) or (a)-(e)) of the steps (a)-(f) with denaturation between repeating cycles. The reaction repetition may be at least five, ten, twenty or thirty times, and no more than seventy, sixty, fifty or forty times.

### Kits for Target Detection

In still another aspect of this invention, there is provided a kit for detecting a target nucleic acid sequence in a nucleic acid sample by a PCE-IH (PTO Cleavage and Extension-Dependent Immobilized Oligonucleotide Hybridization) assay on a solid phase, comprising:
(a) an upstream oligonucleotide; wherein the upstream oligonucleotide comprises a hybridizing nucleotide sequence complementary to the target nucleic acid sequence;
(b) a PTO (Probing and Tagging Oligonucleotide); wherein the PTO comprises (i) a 3'-targeting portion comprising a hybridizing nucleotide sequence complementary to the target nucleic acid sequence and (ii) a 5'-tagging portion comprising a nucleotide sequence non-complementary to the target nucleic acid sequence; wherein the 3'-targeting portion is hybridized with the target nucleic acid sequence and the 5'-tagging portion is not hybridized with the target nucleic acid sequence; the upstream oligonucleotide is located upstream of the PTO; wherein the upstream oligonucleotide or its extended strand induces cleavage of the PTO by the enzyme having the 5' nuclease activity such that the cleavage releases a fragment comprising the 5'-tagging portion or a part of the 5'-tagging portion of the PTO;
(c) a CTO (Capturing and Templating Oligonucleotide); wherein the CTO comprises in a 3' to 5' direction (i) a capturing portion comprising a nucleotide sequence complementary to the 5'-tagging portion or a part of the 5'-tagging portion of the PTO and (ii) a templating portion comprising a nucleotide sequence non-complementary to the 5'-tagging portion and the 3-targeting portion of the PTO; wherein the fragment released from the PTO is hybridized with the capturing portion of the CTO; wherein the fragment hybridized with the capturing portion of the CTO is extended to produce an extended strand containing an extended sequence complementary to the templating portion of the CTO and an extended duplex is formed; wherein the extended strand comprises a label; and
(d) an IO (immobilized oligonucleotide); wherein the IO is immobilized on a solid substrate; wherein the IO comprises a sequence complementary to the extended strand; wherein the hybridization between the extended strand with the IO provides a detectable signal on the solid substrate, wherein the sequence of the IO is selected not to form a hybrid with an undigested PTO.

Since the kit of this invention is constructed to perform the detection method of the present invention described above, the common descriptions between them are omitted in order to avoid undue redundancy leading to the complexity of this specification.

In certain embodiment, the kit further comprises an enzyme having a 5' nuclease activity. In certain embodiment, the enzyme having the 5' nuclease activity is a polymerase having a 5' nuclease activity or FEN nuclease.

According to an embodiment, the PTO and the CTO are contained in a mole ratio of the PTO to the CTO of more than 1 (particularly, the mole ratio of not less than 1.2, not less than 1.3, not less than 1.5, not less than 1.8, not less than 2, not less than 3, not less than 4, not less than 5, not less than 7 or not less than 10).

According to an embodiment, the IO comprises a sequence complementary to (i) all or a part of the extended sequence and (ii) a part of the fragment in the extended strand.

According to an embodiment, the label contained in the extended strand is (i) a single label linked to the fragment released from the PTO, (ii) a label to be incorporated into the extended strand during the extension reaction, or (iii) a combination of the single label linked to the fragment and the label to be incorporated into the extended strand during the extension reaction.

According to an embodiment, the kit further comprises a label to be incorporated into the extended strand during the extension reaction.

In certain embodiment, the label is the label to be incorporated into the extended strand, the templating portion of the CTO comprises a nucleotide containing a first non-natural base, and the extension reaction of the step (d) is performed in the presence of a nucleotide containing a label and a second non-natural base with a specific binding affinity to the first non-natural, thereby incorporating the label into the extended strand.

In certain embodiment, the label is a combination of the single label linked to the fragment and the label to be incorporated into the extended strand during the extension reaction, and the single label and the incorporated label are an interactive dual label containing a pair of an acceptor and a donor.

In certain embodiment, the kit further comprises an intercalating dye and the label contained in the extended strand is an acceptor to receive signal from the intercalating dye.

According to an embodiment, the PTO, the CTO and/or the IO is blocked at its 3'-end to prohibit its extension.

In certain embodiment, the upstream oligonucleotide is an upstream primer or an upstream probe.

According to an embodiment, the capturing portion of the CTO comprises at its 5'-end part a nucleotide sequence complementary to a part of the 3'-targeting portion of the PTO. According to an embodiment, the nucleotide sequence complementary to the part of the 3'-targeting portion of the PTO is 1-10 nucleotides in length.

According to an embodiment, the kit is used for detecting at least two types of target nucleic acid sequences; wherein the upstream oligonucleotide comprises at least two types of oligonucleotides, the PTO comprises at least two types of the PTOs, the CTO comprises at least two types of the CTOs and the IO comprises at least two types of the IOs.

According to an embodiment, the target nucleic acid sequence comprises a nucleotide variation.

According to an embodiment, the kit further comprises a downstream primer.

The features and advantages of this invention will be summarized as follows:
(a) The present invention firstly hybridizes the PTO with a target nucleic acid sequence, forms the extended strand in a target-dependent manner by using the CTO having artificially selected sequence as templates and finally hybridizes the extended strand with the IO immobilized on a solid phase. In other words, the present invention employs a series of reactions including PTO hybridization and cleavage, CTO hybridization and extension and IO hybridization, which is responsible for the highly enhanced specificity of the present invention.
(b) Direct hybridization between a target nucleic acid sequence and an oligonucleotide immobilized on a solid phase is unlikely to effectively produce reaction results because the solid phase may provide restricted reaction environment. Unlikely, the present invention utilizes hybridization between the extended strand and the IO with no involvement of target nucleic acid sequences, such that the detection results may be more effectively produced under general or typical reaction conditions. Such advantages enable to establish reaction conditions for multiplex target detection of clinical samples having a wide variety of characteristics in more convenient manner and to prevent false signal generation.
(c) The IO immobilized on a solid phase comprises no label. Therefore, there is no problem of label degradation being likely to occur in long-term storage of labeled oligonucleotide immobilized on a solid substrate.
(d) In conventional methods using direct hybridization of a probe with a target nucleic acid sequence, the probe binds to the target nucleic acid sequence in a competitive manner with the complementary sequence. In contrast, the present invention may avoid such competitive hybridization. The present invention enables probe hybridization to become more effective because only the extended strand may be amplified using the CTO as templates by adjusting the amounts of the PTO and the CTO, such that the extended strand can be effectively hybridized with the IO with no hindrance of the CTO.
   Furthermore, in the conventional methods using direct hybridization of the probe with one strand of the target nucleic acid sequence, the length of the other strand of the target nucleic acid sequence is relatively longer than that of the probe. Therefore, there is limitation in enhancing hybridization competitivity of the probe over that of the other strand with the probe by using longer probes (i.e., higher Tₘ probes). In the present invention, the length of the CTO inhibiting hybridization between the extended strand and the IO can be adjusted to become shorter. Therefore, the hybridization competitivity of the IO can be enhanced over that of the CTO by adjusting the length of the IO (i.e., adjustment of Tₘ values).
(e) The present invention ensures to detect not only signals on a solid phase in a real-time manner but also signals provided over a range of temperatures (e.g., melting curve analysis).
(f) Because the present invention utilizes oligonucleotides immobilized on a solid phase, it can simultaneously detect a plurality of target nucleic acid sequences even using a single type of a fluorescent label.
(g) It is noteworthy that the sequence of the 5'-tagging portion of PTO, the sequence of CTO and the sequence of the IO can be selected with no consideration of target nucleic acid sequences. This makes it possible to pre-design a pool of sequences for the 5'-tagging portion of PTO, the CTO and the IO. Although the 3'-targeting portion of the PTO has to be prepared with considering target nucleic acid sequences, the CTO and IO can be prepared in a ready-made fashion with no consideration or knowledge of target nucleic acid sequences.

The present invention will now be described in further detail by examples. It would be obvious to those skilled in the art that these examples are intended to be more concretely illustrative and the scope of the present invention as set forth in the appended claims is not limited to or by the examples.

### EXAMPLES

### Example 1: Evaluation of PTO Cleavage and Extension dependent Immobilized Oligonucleotide Hybridization (PCE-IH) assay.

We examined whether PCE-IH assay using the single-labeled PTO (Probing and Tagging Oligonucleotide), CTO (Capturing and Templating Oligonucleotide), and the immobilized oligonucleotide (IO) on microarray can detect a target nucleic acid sequence.

Taq DNA polymerase having a 5' nuclease activity was used for the extension of upstream primer and downstream primer, the cleavage of PTO, and the extension of PTO fragment. Genomic DNA of Neisseria gonorrhoeae (NG) was used as a target nucleic acid sequence.

PTO has a fluorescent reporter molecule (Quasar570) at its 5'-end. PTO, CTO, and IO are blocked with a carbon spacer at their 3'-end. IO has poly(T)₁₀ as a linker arm and was immobilized on the surface of a glass slide by using an amino group (AminnoC6) at its 5'-end. A marker probe having a fluorescent reporter molecule (Quasar570) at its 5'-end was immobilized on the surface of the glass slide by using an amino group (AminnoC7) at its 3'-end.

In this Example, if NG is present, a PTO specifically hybridized to NG is cleaved and a fluorescent reporter-labeled PTO fragment is produced. The PTO fragment is annealed to the capturing portion of the CTO, extended on the templating portion of the CTO and forms an extended strand having a fluorescent reporter molecule at its 5'-end. The extended strand can hybridize to IO. The cleavage of PTO and the extension of PTO fragment, the hybridization of the extended strand to IO were conducted simultaneously on the microarray. After the reaction, the presence or absence of the extended strand-IO duplex was analyzed.

The sequences of upstream primer, downstream primer, PTO, CTO, IO, and marker used in this Example are:
NG-F 5'-TACGCCTGCTACTTTCACGCTIIIIIGTAATCAGATG-3' (SEQ ID NO: 1)
NG-R 5'-CAATGGATCGGTATCACTCGCIIIIICGAGCAAGAAC-3' (SEQ ID NO: 2)
PTO 5'-[Quasar570]ACGACGGCTTGGCTGCCCCTCATTGGCGTGTTTCG[C3 spacer]-3' (SEQ ID NO: 3)
CTO 5'-CATATCGTCCAGGGTATCCAGCGCTCAGCCAAGCCGTCGT[C3 spacer]-3' (SEQ ID NO: 4)
IO 5'-[Amino C6]TTTTTTTTTTCATATCGTCCAGGGTATCCAGCGC[C3 spacer]-3' (SEQ ID NO: 5)
Marker 5'-[Quasar570]ATATATATAT[Amino C7J-3' (SEQ ID NO: 6)
(I: Deoxyinosine)
(Underlined letters indicate the 5'-tagging portion of PTO)

NSB9 NHS slides (NSBPOSTECH, Korea) were used for fabrication of the IO and marker (SEQ ID NOs: 5 and 6). The IO and marker dissolved in NSB spotting buffer at the final concentration of 50 µM were printed on the NSB9 NHS slides with PersonalArrayer^{™}16 Microarray Spotter (CapitalBio, China). The IO and marker were spotted side by side in a 3x1 format (triplicate spots), and the resulting microarray was incubated in a chamber maintained at ~85% humidity for overnight. The slides were then washed in a buffer solution containing 2×SSPE (0.3 M sodium chloride, 0.02 M sodium hydrogen phosphate and 2.0 mM EDTA), pH 7.4 and 7.0 mM SDS at 37°C for 30 min to remove the non-specifically bound IO and marker and rinsed with distilled water. Then, the DNA-functionalized slides were dried using a slide centrifuge and stored in dark at 4°C until use.

The PCE-IH reaction was conducted in the final volume of 30 µl containing 100 pg of genomic DNA of NG gene, 10 pmole of upstream primer (SEQ ID NO: 1), 10 pmole of downstream primer (SEQ ID NO: 2), 5 pmole of PTO (SEQ ID NO: 3), 0.5 pmole of CTO (SEQ ID NO: 4) and 15 µl of 2X Master Mix [containing 2.5 mM MgCl₂, 200 µM of dNTPs, and 2.4 units of H-Taq DNA polymerase (Solgent, Korea)]; the whole mixture was applied to a chamber assembled on the surface of NSB glass slide on which the IO (SEQ ID NO: 5) was cross-linked. The slide was placed on in situ block in a thermocycler (GenePro B4I, China). The PCE-IH reaction was carried out as follows: 15 min denaturation at 95°C and 40 cycles of 30 sec at 95°C, 60 sec at 60°C, 30 sec at 72°C and 30 min hybridization at 55°C.

After the reaction, the slides were washed in distilled water for 1 min. The image acquisition was carried out by the use of Confocal Laser Scanner, Axon GenePix4300A (Molecular Device, US) with scanning at 5-µm pixel resolution. The fluorescence intensity was analyzed by the use of quantitative microarray analysis software, GenePix pro7.0 software (Molecular Device, US). The fluorescence intensity was expressed as spot-medians after local background subtractions. Each spot was triplicated for the test of reproducibility. The fluorescence intensity indicates the average value of the triplicated spots.

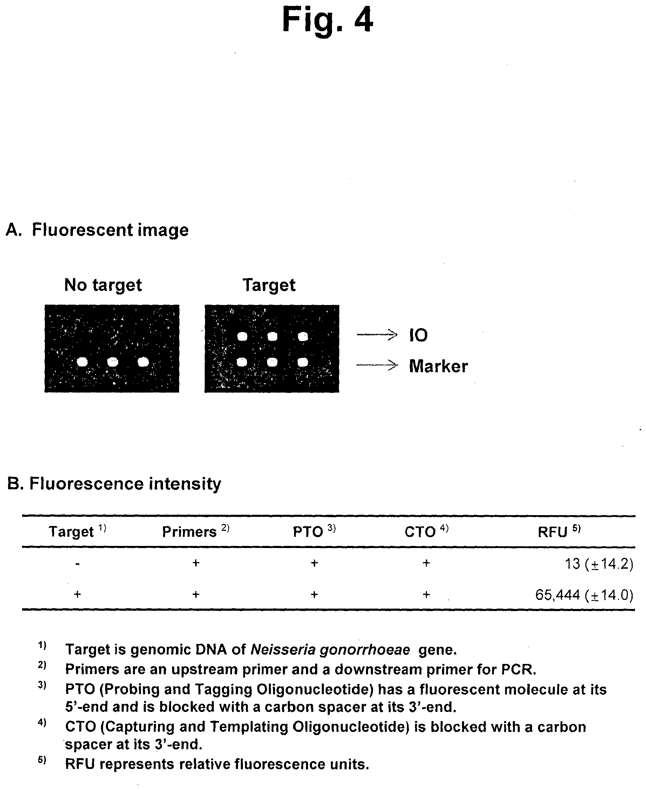

As shown in Figure 4, the fluorescent intensity was apparently increased in the presence of the target in comparison to that in the absence of the target. These results show that PCE-IH assay using the single-labeled PTO, CTO, and IO on microarray can detect a target nucleic acid sequence.

### Example 2: Evaluation of Effect of CTO on Hybridization of IO with Extended Strand.

When an extended strand is generated in the presence of the target nucleic acid sequence, the formation of the hybrid between the extended strand and the IO can be inhibited by the hybridization of the extended strand with the CTO. We investigated the effect of the CTO on the formation of the hybrid between the extended strand and the IO by using various concentrations of the CTO and synthetic extended strand (Syn-ES) which imitates the extended strand. Syn-ES has a fluorescent reporter molecule (Quasar570) at its 5'-end.

The same Taq DNA polymerase, CTO, IO and marker used in Example 1 were used in this Example

The sequence of Syn-ES used in this Example is:
Syn-ES 5'-[Quasar570]ACGACGGCTTGGCTGAGCGCTGGATACCCTGGACGATATG-3' (SEQ ID NO: 7)

Slide preparation was conducted as the same protocol used in Example 1.

The reaction was conducted in the final volume of 30 µl containing the CTO (2, 1, 0.5, or 0 pmole) (SEQ ID NO: 4), 1 pmole of Syn-ES (SEQ ID NO: 7), and 15 µl of 2X Master Mix [containing 2.5 mM MgCl₂, 200 µM of dNTPs, and 2.4 units of H-Taq DNA polymerase (Solgent, Korea)]; the whole mixture was applied to a chamber assembled on the surface of NSB glass slide on which the IO (SEQ ID NO: 5) was cross-linked. The slide was placed on in situ block in a thermocycler (GenePro B4I, China). The hybridization reaction was performed at 55°C for 30 min.

After the reaction, the slides were washed in distilled water for 1 min. The image acquisition was carried out by the use of Confocal Laser Scanner, Axon GenePix4300A (Molecular Device, US) with scanning at 5-µm pixel resolution. The fluorescence intensity was analyzed by the use of quantitative microarray analysis software, GenePix pro7.0 software (Molecular Device, US). The fluorescence intensity was expressed as spot-medians after local background subtractions. Each spot was triplicated for the test of reproducibility. The fluorescence intensity indicates the average value of the triplicated spots.

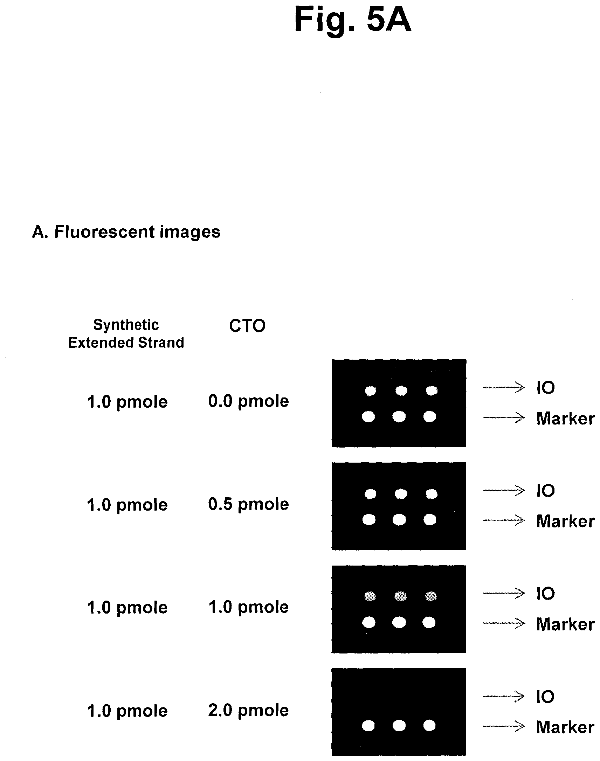

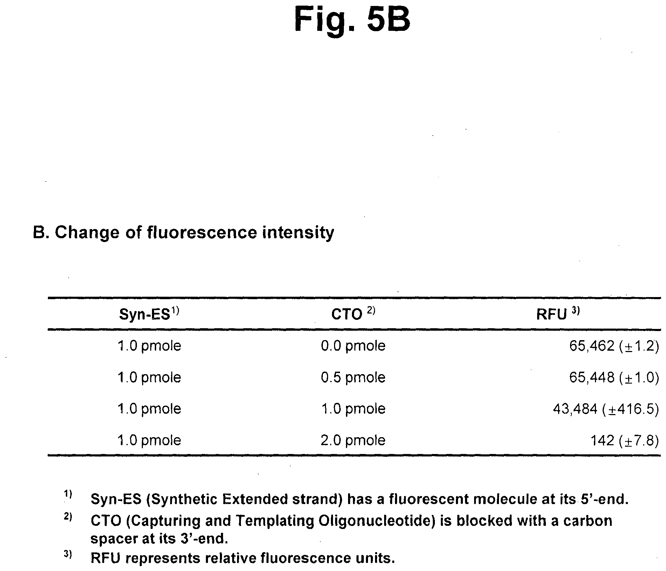

As shown in Figures 5a and 5b, the fluorescent intensity was apparently decreased as the concentration of CTO increased.

### Example 3: Evaluation of PCE-IH assay with variation of ratio of PTO and CTO.

As shown in Example 2, the formation of the hybrid between the extended strand and the CTO inhibits the hybridization of the extended strand to the IO. When an extended strand is generated in the presence of the target nucleic acid sequence, an increase in the number of the extended strand in single-stranded state enhances the hybridization of the extended strand to the IO.

One way to regulate the number of the extended strand in single-stranded state is varying ratio of the PTO and the CTO. In the presence of the target, the use of a higher concentration of the PTO over the CTO increases the generation of the extended strand in single-stranded state, leading to improving the fluorescent intensity. We examined how the variation of ratio of PTO and CTO influences the fluorescent intensity by using various concentrations of the CTO.

The same Taq DNA polymerase, upstream primer, downstream primer, PTO, CTO, IO and marker used in Example 1 were used in this Example.

Slide preparation was conducted as the same protocol used in Example 1.

The PCE-IH reaction was conducted in the final volume of 30 µl containing 100 pg of genomic DNA of NG gene, 10 pmole of upstream primer (SEQ ID NO: 1), 10 pmole of downstream primer (SEQ ID NO: 2), 5 pmole of PTO (SEQ ID NO: 3), the CTO (3, 1, or 0.5 pmole) (SEQ ID NO: 4) and 15 µl of 2X Master Mix [containing 2.5 mM MgCl₂, 200 µM of dNTPs, and 2.4 units of H-Taq DNA polymerase (Solgent, Korea)]; the whole mixture was applied to a chamber assembled on the surface of NSB glass slide on which the IO (SEQ ID NO: 5) was cross-linked. The slide was placed on in situ block in a thermocycler (GenePro B4I, China). The PCE-IH reaction was carried out as follows: 15 min denaturation at 95°C and 40 cycles of 30 sec at 95°C, 60 sec at 60°C, 30 sec at 72°C and 30 min hybridization at 55°C.

After the reaction, the slides were washed in distilled water for 1 min. The image acquisition was carried out by the use of Confocal Laser Scanner, Axon GenePix4300A (Molecular Device, US) with scanning at 5-µm pixel resolution. The fluorescence intensity was analyzed by the use of quantitative microarray analysis software, GenePix pro7.0 software (Molecular Device, US). The fluorescence intensity was expressed as spot-medians after local background subtractions. Each spot was triplicated for the test of reproducibility. The fluorescence intensity indicates the average value of the triplicated spots.

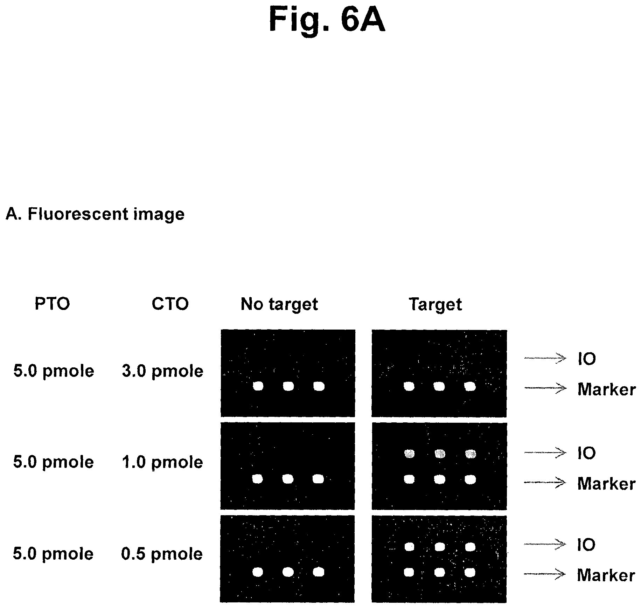

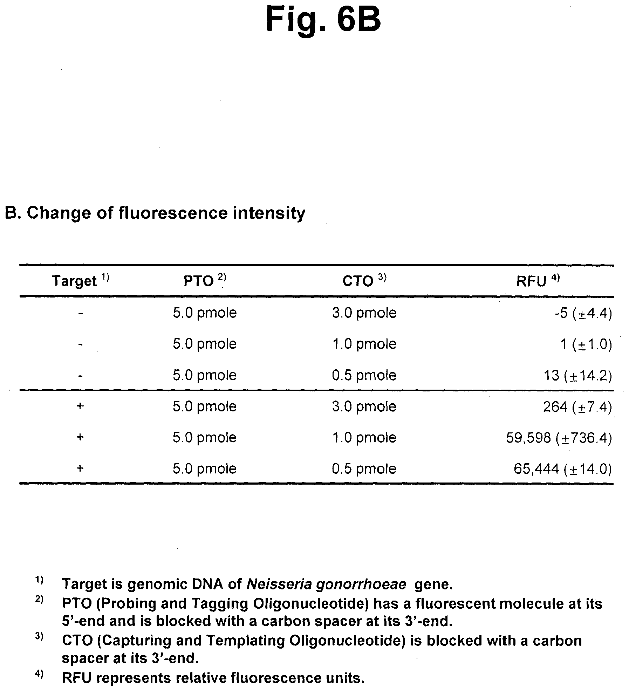

As shown in Figures 6a and 6b, according to the decrease in concentration of CTO, the fluorescent intensity was apparently increased in the presence of the target.

The scope of this invention is to be determined by appended claims and their equivalents.

## Claims

1. A method for detecting a target nucleic acid sequence in a nucleic acid sample by a PCE-IH (PTO Cleavage and Extension-Dependent Immobilized Oligonucleotide Hybridization) assay on a solid phase, comprising:
(a) hybridizing the target nucleic acid sequence with a PTO (Probing and Tagging Oligonucleotide); wherein the PTO comprises (i) a 3-targeting portion comprising a hybridizing nucleotide sequence complementary to the target nucleic acid sequence and (ii) a 5'-tagging portion comprising a nucleotide sequence non-complementary to the target nucleic acid sequence; wherein the 3-targeting portion is hybridized with the target nucleic acid sequence and the 5'-tagging portion is not hybridized with the target nucleic acid sequence;
(b) contacting the resultant of the step (a) to the enzyme having a 5' nuclease activity under conditions for cleavage of the PTO;
wherein the PTO is cleaved by the enzyme having the 5' nuclease activity such that the cleavage releases a fragment comprising the 5'-tagging portion or a part of the 5'-tagging portion of the PTO;
(c) hybridizing the fragment released from the PTO with a CTO (Capturing and Templating Oligonucleotide); wherein the CTO comprises in a 3'to 5' direction (i) a capturing portion comprising a nucleotide sequence complementary to the 5'-tagging portion or a part of the 5'-tagging portion of the PTO and (ii) a templating portion comprising a nucleotide sequence non-complementary to the 5'-tagging portion and the 3'-targeting portion of the PTO; wherein the fragment released from the PTO is hybridized with the capturing portion of the CTO;
(d) performing an extension reaction using the resultant of the step (c) and a template-dependent nucleic acid polymerase; wherein the fragment hybridized with the capturing portion of the CTO is extended to produce an extended strand containing an extended sequence complementary to the templating portion of the CTO and an extended duplex is formed; wherein the extended strand comprises a label;
(e) hybridizing the extended strand with an IO (immobilized oligonucleotide) immobilized on a solid substrate; wherein the IO comprises a sequence complementary to the extended strand; wherein the hybridization between the extended strand with the IO provides a detectable signal on the solid substrate; wherein the sequence of the IO is selected not to form a hybrid with an undigested PTO and
(f) detecting the signal indicative of the presence of the extended strand; the presence of the extended strand indicates the presence of the target nucleic acid sequence.

2. The method according to claim 1, wherein the method is performed in the presence of an upstream oligonucleotide; wherein the upstream oligonucleotide comprises a hybridizing nucleotide sequence complementary to the target nucleic acid sequence and the upstream oligonucleotide is located upstream of the PTO; and wherein the upstream oligonucleotide or its extended strand induces cleavage of the PTO by the enzyme having the 5' nuclease activity.

3. The method according to claim 1 or 2, wherein the step (e) is performed under conditions favorable for hybridization of the extended strand through its single-stranded state with the IO, whereby inhibition of the hybridization between the extended strand and the IO by the formation of the extended duplex between the extended strand and the CTO is prevented.

4. The method according to claim 3, wherein the conditions permit to increase the number of the extended strand in the single-stranded state in the step (e).

5. The method according to claim 4, wherein the conditions permitting to increase the number of the extended strand in the single-stranded state in the step (e) are denaturation of the extended duplex prior to the hybridization between the extended strand and the IO.

6. The method according to claim 4, wherein the conditions permitting to increase the number of the extended strand in the single-stranded state in the step (e) are removal of the CTO prior to the hybridization between the extended strand and the IO.

7. The method according to claim 4, wherein the conditions permitting to increase the number of the extended strand in the single-stranded state in the step (e) are to use the PTO and the CTO in a mole ratio of the PTO to the CTO of more than 1.

8. The method according to claim 7, wherein the mole ratio of the PTO to the CTO is not less than 1.2.

9. The method according to claim 1, where the step (e) is performed under conditions enhancing a hybridization competitivity of the extended strand with the IO over that of the CTO with the extended strand.

10. The method according to claim 9, wherein the conditions permitting to enhance the hybridization competitivity of the extended strand with the IO over that of the CTO with the extended strand are to adjust Tₘ value of a hybrid between the extended strand and the IO and Tₘ value of a hybrid between the CTO and the extended strand.

11. The method according to claim 1, wherein the IO comprises a sequence complementary to (i) all or a part of the extended sequence and (ii) a part of the fragment in the extended strand.

12. The method according to claim 1, wherein the label contained in the extended strand is (i) a single label linked to the fragment released from the PTO, (ii) a label to be incorporated into the extended strand during the extension reaction, or (iii) a combination of the single label linked to the fragment and the label to be incorporated into the extended strand during the extension reaction.

13. The method according to claim 12, wherein the label is the label to be incorporated into the extended strand, the templating portion of the CTO comprises a nucleotide containing a first non-natural base, and the extension reaction of the step (d) is performed in the presence of a nucleotide containing a label and a second non-natural base with a specific binding affinity to the first non-natural, thereby incorporating the label into the extended strand.

14. The method according to claim 12, wherein the label is a combination of the single label linked to the fragment and the label to be incorporated into the extended strand during the extension reaction, and the single label and the incorporated label are an interactive dual label containing a pair of an acceptor and a donor.

15. The method according to claim 12, wherein the hybridization of the step (e) is performed in the presence of an intercalating dye, the label contained in the extended strand is an acceptor to receive signal from the intercalating dye, the signal from the intercalating dye intercalated into a hybrid between the extended strand and the IO is transferred to the acceptor, and the acceptor provides the detectable signal.

16. The method according to claim 1, wherein the PTO, the CTO and/or the IO is blocked at its 3'-end to prohibit its extension.

17. The method according to claim 1, wherein the step (f) for detecting the signal is performed by detecting a signal on the solid substrate provided in a process of melting a hybrid between the extended strand and the IO over a range of temperatures or melting and hybridizing the hybrid.

18. The method according to claim 1, wherein the method further comprises repeating all or some of the steps (a)-(f) with denaturation between repeating cycles.

19. The method according to claim 1, wherein the steps (a)-(f) are performed in a reaction vessel or in separate reaction vessels.

20. The method according to claim 1, wherein the method is performed to detect at least two types of target nucleic acid sequences; wherein the upstream oligonucleotide comprises at least two types of oligonucleotides, the PTO comprises at least two types of the PTOs, the CTO comprises at least two types of the CTOs and the IO comprises at least two types of the IOs.

21. The method according to claim 1, wherein the target nucleic acid sequence comprises a nucleotide variation.

22. The method according to any one of claims 1-21, wherein the method is performed in the presence of a downstream primer.

23. A kit for detecting a target nucleic acid sequence in a nucleic acid sample by a PCE-IH (PTO Cleavage and Extension-Dependent Immobilized Oligonucleotide Hybridization) assay on a solid phase comprising:
(a) an upstream oligonucleotide; wherein the upstream oligonucleotide comprises a hybridizing nucleotide sequence complementary to the target nucleic acid sequence;
(b) a PTO (Probing and Tagging Oligonucleotide); wherein the PTO comprises (i) a 3'-targeting portion comprising a hybridizing nucleotide sequence complementary to the target nucleic acid sequence and (ii) a 5'-tagging portion comprising a nucleotide sequence non-complementary to the target nucleic acid sequence; wherein the 3'-targeting portion is hybridized with the target nucleic acid sequence and the 5'-tagging portion is not hybridized with the target nucleic acid sequence; the upstream oligonucleotide is located upstream of the PTO; wherein the upstream oligonucleotide or its extended strand induces cleavage of the PTO by the enzyme having the 5' nuclease activity such that the cleavage releases a fragment comprising the 5'-tagging portion or a part of the 5'-tagging portion of the PTO;
(c) a CTO (Capturing and Templating Oligonucleotide); wherein the CTO comprises in a 3' to 5' direction (i) a capturing portion comprising a nucleotide sequence complementary to the 5'-tagging portion or a part of the 5'-tagging portion of the PTO and (ii) a templating portion comprising a nucleotide sequence non-complementary to the 5'-tagging portion and the 3-targeting portion of the PTO; wherein the fragment released from the PTO is hybridized with the capturing portion of the CTO; wherein the fragment hybridized with the capturing portion of the CTO is extended to produce an extended strand containing an extended sequence complementary to the templating portion of the CTO and an extended duplex is formed; wherein the extended strand comprises a label; and
(d) an IO (immobilized oligonucleotide); wherein the IO is immobilized on a solid substrate; wherein the IO comprises a sequence complementary to the extended strand; wherein the hybridization between the extended strand with the IO provides a detectable signal on the solid substrate; wherein the sequence of the IO is selected not to form a hybrid with an undigested PTO.

24. The kit according to claim 23, wherein the PTO and the CTO are contained in a mole ratio of the PTO to the CTO of more than 1.

## Patentansprüche

1. Verfahren zum Detektieren einer Zielnukleinsäuresequenz in einer Nukleinsäureprobe durch einen PCE-IH (PTO Cleavage und Extension-abhängige Immobilisierte Oligonukleotid-Hybridisierung) Assay auf einer festen Phase, umfassend:
(a) Hybridisieren der Zielnukleinsäuresequenz mit einem PTO (Probing und Tagging Oligonukleotid); wobei das PTO umfasst (i) einen 3'-Zielanteil, umfassend eine hybridisierende Nukleotidsequenz, die komplementär ist zu der Zielnukleinsäuresequenz, und (ii) einen 5'-Tagging-Anteil, umfassend eine Nukleotidsequenz, die nicht-komplementär ist zu der Zielnukleinsäuresequenz; wobei der 3'-Zielanteil mit der Zielnukleinsäuresequenz hybridisiert wird und der 5'-Tagging-Anteil mit der Zielnukleinsäuresequenz nicht hybridisiert wird;
(b) In-Kontakt-Bringen des Ergebnisses aus Schritt (a) mit dem Enzym, das eine 5'-Nukleaseaktivität hat, unter Bedingungen für ein Spalten des PTO;
wobei das PTO gespalten wird durch das Enzym, das die 5'-Nukleaseaktivität hat, so dass die Spaltung ein Fragment freisetzt, umfassend den 5'-Tagging-Anteil oder einen Teil des 5'-Tagging-Anteils des PTO;
(c) Hybridisieren des vom PTO freigesetzten Fragments mit einem CTO (Capturing und Templating Oligonukleotid); wobei das CTO umfasst in einer 3' zu 5' Richtung (i) einen Capturing-Anteil, umfassend eine Nukleotidsequenz, die komplementär ist zu dem 5'-Tagging-Anteil oder einem Teil des 5'-Tagging-Anteils des PTO, und (ii) einen Templating-Anteil, umfassend eine Nukleotidsequenz, die nicht-komplementär ist zu dem 5'-Tagging-Anteil und dem 3'-Zielanteil des PTO; wobei das vom PTO freigesetzte Fragment mit dem Capturing-Anteil des CTO hybridisiert wird;
(d) Durchführen einer Verlängerungsreaktion unter Verwendung des Ergebnisses aus Schritt (c) und einer Template-abhängigen Nukleinsäurepolymerase; wobei das Fragment, das mit dem Capturing-Anteil des CTO hybridisiert ist, verlängert wird, um einen verlängerten Strang zu produzieren, der eine verlängerte Sequenz enthält, die komplementär ist zu dem Templating-Anteil des CTO und ein verlängerter Duplex gebildet wird; wobei der verlängerte Strang ein Label umfasst;
(e) Hybridisieren des verlängerten Strangs mit einem IO (Immobilisiertes Oligonukleotid), das auf einem festen Substrat immobilisiert ist; wobei das IO eine Sequenz umfasst, die komplementär ist zu dem verlängerten Strang; wobei die Hybridisierung zwischen dem verlängerten Strang mit dem IO ein detektierbares Signal auf dem festen Substrat bereitstellt; wobei die Sequenz des IO so gewählt ist, dass sie nicht ein Hybrid mit einem unverdauten PTO bildet und
(f) Nachweisen des Signals, das auf das Vorhandensein des verlängerten Strangs hinweist; das Vorhandensein des verlängerten Strangs weist auf das Vorhandensein der Zielnukleinsäuresequenz hin.

2. Verfahren nach Anspruch 1, wobei das Verfahren in der Anwesenheit eines Upstream-Oligonukleotids durchgeführt wird; wobei das Upstream-Oligonukleotid eine hybridisierende Nukleotidsequenz umfasst, die zu der Zielnukleinsäuresequenz komplementär ist, und das Upstream-Oligonukleotid sich upstream des PTO befindet; und wobei das Upstream-Oligonukleotid oder dessen verlängerter Strang ein Spalten des PTO induziert durch das Enzym, das die 5'-Nukleaseaktivität hat.

3. Verfahren nach Anspruch 1 oder 2, wobei Schritt (e) unter Bedingungen durchgeführt wird, die vorteilhaft sind für eine Hybridisierung des verlängerten Strangs durch dessen einzelsträngigen Zustand mit dem IO, wobei eine Inhibition der Hybridisierung zwischen dem verlängerten Strang und dem IO durch die Bildung des verlängerten Duplex zwischen dem verlängerten Strang und dem CTO verhindert wird.

4. Verfahren nach Anspruch 3, wobei die Bedingungen es erlauben, die Anzahl des verlängerten Strangs im einzelsträngigen Zustand in Schritt (e) zu erhöhen.

5. Verfahren nach Anspruch 4, wobei die Bedingungen, welche es erlauben, die Anzahl des verlängerten Strangs im einzelsträngigen Zustand in Schritt (e) zu erhöhen, eine Denaturierung des verlängerten Duplex vor der Hybridisierung zwischen dem verlängerten Strang und dem IO sind.

6. Verfahren nach Anspruch 4, wobei die Bedingungen, welche es erlauben, die Anzahl des verlängerten Strangs im einzelsträngigen Zustand in Schritt (e) zu erhöhen, ein Entfernen des CTO vor der Hybridisierung zwischen dem verlängerten Strang und dem IO sind.

7. Verfahren nach Anspruch 4, wobei die Bedingungen, welche es erlauben, die Anzahl des verlängerten Strangs im einzelsträngigen Zustand in Schritt (e) zu erhöhen, ein Verwenden des PTO und des CTO in einem Molverhältnis des PTO zu dem CTO von mehr als 1 sind.

8. Verfahren nach Anspruch 7, wobei das Molverhältnis des PTO zu dem CTO nicht weniger als 1,2 ist.

9. Verfahren nach Anspruch 1, bei dem Schritt (e) unter Bedingungen durchgeführt wird, welche eine Hybridisierungskompetitivität des verlängerten Strangs mit dem IO gegenüber derjenigen des CTO mit dem verlängerten Strang erhöhen.

10. Verfahren nach Anspruch 9, wobei die Bedingungen, die ein Erhöhen der Hybridisierungskompetitivität des verlängerten Strangs mit dem IO gegenüber derjenigen des CTO mit dem verlängerten Strang erlauben, ein Anpassen eines Tₘ-Werts eines Hybrids zwischen dem verlängerten Strang und dem IO und eines Tₘ-Werts eines Hybrids zwischen dem CTO und dem verlängerten Strang sind.

11. Verfahren nach Anspruch 1, wobei das IO eine Sequenz umfasst, die komplementär ist zu (i) der gesamten oder einem Teil der verlängerten Sequenz und (ii) einem Teil des Fragments im verlängerten Strang.

12. Verfahren nach Anspruch 1, wobei das Label, das im verlängerten Strang enthalten ist, (i) ein einzelnes Label, verbunden mit dem vom PTO freigesetzten Fragment, (ii) ein während der Verlängerungsreaktion in den verlängerten Strang zu integrierendes Label, oder (iii) eine Kombination aus dem einzelnen Label, verbunden mit dem Fragment, und dem während der Verlängerungsreaktion in den verlängerten Strang zu integrierenden Label, ist.

13. Verfahren nach Anspruch 12, wobei das Label das während der Verlängerungsreaktion in den verlängerten Strang zu integrierende Label ist, der Templating-Anteil des CTO ein Nukleotid umfasst, welches eine erste nicht-natürliche Base enthält, und die Verlängerungsreaktion von Schritt (d) durchgeführt wird in der Anwesenheit eines Nukleotids, beinhaltend ein Label und eine zweite nicht-natürliche Base mit einer spezifischen Bindungsaffinität zu der ersten nicht-natürlichen Base, wodurch das Label in den verlängerten Strang einbaut wird.

14. Verfahren nach Anspruch 12, wobei das Label eine Kombination ist aus dem einzelnen Label, verbunden mit dem Fragment, und dem während der Verlängerungsreaktion in den verlängerten Strang zu integrierenden Label, und das einzelne Label und das integrierte Label ein interaktives Dual-Label sind, welches ein Paar aus einem Akzeptor und einem Donor enthält.

15. Verfahren nach Anspruch 12, wobei die Hybridisierung von Schritt (e) durchgeführt wird in der Anwesenheit eines interkalierenden Farbstoffs, das Label, das im verlängerten Strang enthalten ist, ein Akzeptor ist, um Signal vom interkalierenden Farbstoff zu empfangen, das Signal vom interkalierenden Farbstoff, welcher in ein Hybrid zwischen dem verlängerten Strang und dem IO interkaliert, auf den Akzeptor übertragen wird, und der Akzeptor das detektierbare Signal bereitstellt.

16. Verfahren nach Anspruch 1, wobei das PTO, das CTO und/oder das IO an seinem 3'-Ende blockiert ist, um seine Verlängerung zu unterbinden.

17. Verfahren nach Anspruch 1, wobei Schritt (f) für das Detektieren des Signals durchgeführt wird, durch das Detektieren eines Signals auf dem festen Substrat, das bereitgestellt wird in einem Prozess des Schmelzens eines Hybrids zwischen dem verlängerten Strang und dem IO über einen Temperaturbereich hinweg, oder des Schmelzens und Hybridisierens des Hybrids.

18. Verfahren nach Anspruch 1, wobei das Verfahren weiterhin umfasst das Wiederholen von allen oder einigen der Schritte (a)-(f) mit Denaturierung zwischen sich wiederholenden Zyklen.

19. Verfahren nach Anspruch 1, wobei die Schritte (a)-(f) in einem Reaktionsgefäß oder in separaten Reaktionsgefäßen durchgeführt werden.

20. Verfahren nach Anspruch 1, wobei das Verfahren durchgeführt wird, um mindestens zwei Arten von Zielnukleinsäuresequenzen zu detektieren; wobei das Upstream-Oligonukleotid mindestens zwei Arten von Oligonukleotiden umfasst, das PTO mindestens zwei Arten der PTOs umfasst, das CTO mindestens zwei Arten der CTOs umfasst und das IO mindestens zwei Arten der IOs umfasst.

21. Verfahren nach Anspruch 1, wobei die Zielnukleinsäuresequenz eine Nukleotidvariation umfasst.

22. Verfahren nach einem der Ansprüche 1-21, wobei das Verfahren durchgeführt wird in der Anwesenheit eines Downstream-Primers.

23. Kit zum Detektieren einer Zielnukleinsäuresequenz in einer Nukleinsäureprobe durch einen PCE-IH (PTO Cleavage und Extension-abhängige Immobilisierte Oligonukleotid-Hybridisierung) Assay auf einer festen Phase umfassend:
(a) ein Upstream-Oligonukleotid; wobei das Upstream-Oligonukleotid eine hybridisierende Nukleotidsequenz umfasst, die zu der Zielnukleinsäuresequenz komplementär ist;
(b) ein PTO (Probing und Tagging Oligonukleotid); wobei das PTO umfasst (i) einen 3'-Zielanteil, umfassend eine hybridisierende Nukleotidsequenz, die zu der Zielnukleinsäuresequenz komplementär ist, und (ii) einen 5'-Tagging-Anteil, umfassend eine Nukleotidsequenz, die nicht-komplementär ist zu der Zielnukleinsäuresequenz; wobei der 3'-Zielanteil mit der Zielnukleinsäuresequenz hybridisiert wird und der 5'-Tagging-Anteil nicht mit der Zielnukleinsäuresequenz hybridisiert wird; das Upstream-Oligonukleotid sich upstream des PTO befindet; wobei das Upstream-Oligonukleotid oder dessen verlängerter Strang das Spalten des PTO induziert durch das Enzym, das die 5'-Nukleaseaktivität hat, so dass die Spaltung ein Fragment freisetzt, umfassend den 5'-Tagging-Anteil oder einen Teil des 5'-Tagging-Anteils des PTO;
(c) ein CTO (Capturing und Templating Oligonukleotid); wobei das CTO umfasst in einer 3' zu 5' Richtung (i) einen Capturing-Anteil, umfassend eine Nukleotidsequenz, die zu dem 5'-Tagging-Anteil oder einem Teil des 5'-Tagging-Anteils des PTO komplementär ist, und (ii) einen Templating-Anteil, umfassend eine Nukleotidsequenz, die zu dem 5'-Tagging-Anteil und dem 3'-Zielanteil des PTO nicht-komplementär ist; wobei das vom PTO freigesetzte Fragment mit dem Capturing-Anteil des CTO hybridisiert wird; wobei das Fragment, das mit dem Capturing-Anteil des CTO hybridisiert ist, verlängert wird, um einen verlängerten Strang zu produzieren, der eine verlängerte Sequenz enthält, die zu dem Templating-Anteil des CTO komplementär ist, und ein verlängerter Duplex gebildet wird; wobei der verlängerte Strang ein Label umfasst; und
(d) ein IO (Immobilisiertes Oligonukleotid); wobei das IO auf einem festen Substrat immobilisiert ist; wobei das IO eine Sequenz umfasst, die komplementär ist zu dem verlängerten Strang; wobei die Hybridisierung zwischen dem verlängerten Strang mit dem IO ein detektierbares Signal auf dem festen Substrat bereitstellt; wobei die Sequenz des IO so gewählt ist, dass sie nicht ein Hybrid mit einem unverdauten PTO bildet.

24. Kit nach Anspruch 23, wobei das PTO und das CTO in einem Molverhältnis des PTO zu dem CTO von mehr als 1 enthalten sind.

## Revendications

1. Procédé de détection d'une séquence d'acide nucléique cible dans un échantillon d'acide nucléique par un essai de PCE-IH (hybridation d'oligonucléotide immobilisé par clivage et extension de PTO) sur une phase solide, comprenant :
(a) l'hybridation de la séquence d'acide nucléique cible avec un PTO (oligonucléotide de sondage et de marquage) ; dans lequel le PTO comprend (i) une partie de ciblage en 3' comprenant une séquence de nucléotides s'hybridant complémentaire à la séquence d'acide nucléique cible et (ii) une partie de marquage en 5' comprenant une séquence de nucléotides non complémentaire à la séquence d'acide nucléique cible ; dans lequel la partie de ciblage en 3' est hybridée avec la séquence d'acide nucléique cible et la partie de marquage en 5' n'est pas hybridée avec la séquence d'acide nucléique cible ;
(b) la mise en contact du produit résultant de l'étape (a) avec l'enzyme ayant une activité nucléase en 5' dans des conditions de clivage du PTO ;
dans lequel le PTO est clivé par l'enzyme ayant l'activité nucléase en 5' de sorte que le clivage libère un fragment comprenant la partie de marquage en 5' ou une partie de la partie de marquage en 5' du PTO ;
(c) l'hybridation du fragment libéré du PTO avec un CTO (oligonucléotide de capture et de matrice) ; dans lequel le CTO comprend, dans une direction 3' vers 5', (i) une partie de capture comprenant une séquence de nucléotides complémentaire à la partie de marquage en 5' ou à une partie de la partie de marquage en 5' du PTO et (ii) une partie de matrice comprenant une séquence de nucléotides non complémentaire à la partie de marquage en 5' et à la partie de ciblage en 3' du PTO ; dans lequel le fragment libéré du PTO est hybridé avec la partie de capture du CTO ;
(d) la réalisation d'une réaction d'extension en utilisant le produit résultant de l'étape (c) et une polymérase d'acide nucléique dépendante d'une matrice ; dans lequel le fragment hybridé avec la partie de capture du CTO est étendue afin de produire un brin étendu contenant une séquence étendue complémentaire à la partie de matrice du CTO et un duplex étendu est formé ; dans lequel le brin étendu comprend un marqueur ;
(e) l'hybridation du brin étendu avec un IO (oligonucléotide immobilisé) immobilisé sur un substrat solide ; dans lequel l'IO comprend une séquence complémentaire au brin étendu ; dans lequel l'hybridation entre le brin étendu avec l'IO fournit un signal détectable sur le substrat solide ; dans lequel la séquence de l'IO est sélectionnée pour ne pas former d'hybride avec un PTO non digéré et
(f) la détection du signal indiquant la présence du brin étendu ; la présence du brin étendu indiquant la présence de la séquence d'acide nucléique cible.

2. Procédé selon la revendication 1, dans lequel le procédé est réalisé en présence d'un oligonucléotide en amont ; dans lequel l'oligonucléotide en amont comprend une séquence de nucléotides s'hybridant complémentaire à la séquence d'acide nucléique cible et l'oligonucléotide en amont est localisé en amont du PTO ; et dans lequel l'oligonucléotide en amont ou son brin étendu induit le clivage du PTO par l'enzyme ayant l'activité nucléase en 5'.

3. Procédé selon la revendication 1 ou 2, dans lequel l'étape (e) est réalisée dans des conditions favorables à une hybridation du brin étendu, dans son état simple brin, avec l'IO, moyennant quoi une inhibition de l'hybridation entre le brin étendu et l'IO par la formation du duplex étendu entre le brin étendu et le CTO est empêchée.

4. Procédé selon la revendication 3, dans lequel les conditions permettent d'augmenter le nombre du brin étendu dans l'état simple brin à l'étape (e).

5. Procédé selon la revendication 4, dans lequel les conditions permettant d'augmenter le nombre du brin étendu dans l'état simple brin à l'étape (e) sont une dénaturation du duplex étendu avant l'hybridation entre le brin étendu et l'IO.

6. Procédé selon la revendication 4, dans lequel les conditions permettant d'augmenter le nombre du brin étendu dans l'état simple brin à l'étape (e) sont un retrait du CTO avant l'hybridation entre le brin étendu et l'IO.

7. Procédé selon la revendication 4, dans lequel les conditions permettant d'augmenter le nombre du brin étendu dans l'état simple brin à l'étape (e) sont l'utilisation du PTO et du CTO selon un rapport molaire du PTO sur le CTO supérieur à 1.

8. Procédé selon la revendication 7, dans lequel le rapport molaire du PTO sur le CTO n'est pas inférieur à 1,2.

9. Procédé selon la revendication 1, dans lequel l'étape (e) est réalisée dans des conditions améliorant une compétitivité d'hybridation du brin étendu avec l'IO par rapport à celle du CTO avec le brin étendu.

10. Procédé selon la revendication 9, dans lequel les conditions permettant d'augmenter la compétitivité d'hybridation du brin étendu avec l'IO par rapport à celle du CTO avec le brin étendu sont l'ajustement de la valeur Tₘ d'un hybride entre le brin étendu et l'IO et de la valeur Tₘ d'un hybride entre le CTO et le brin étendu.

11. Procédé selon la revendication 1, dans lequel l'IO comprend une séquence complémentaire à (i) l'intégralité ou une partie de la séquence étendue et (ii) une partie du fragment dans le brin étendu.

12. Procédé selon la revendication 1, dans lequel le marqueur contenu dans le brin étendu est (i) un marqueur individuel lié au fragment libéré du PTO, (ii) un marqueur devant être incorporé dans le brin étendu pendant la réaction d'extension, ou (iii) une combinaison du marqueur individuel lié au fragment et du marqueur devant être incorporé dans le brin étendu pendant la réaction d'extension.

13. Procédé selon la revendication 12, dans lequel le marqueur est le marqueur devant être incorporé dans le brin étendu, la partie de matrice du CTO comprend un nucléotide contenant une première base non naturelle, et la réaction d'extension de l'étape (d) est réalisée en présence d'un nucléotide contenant un marqueur et d'une seconde base non naturelle possédant une affinité de liaison spécifique pour la première base non naturelle, en incorporant ainsi le marqueur dans le brin étendu.

14. Procédé selon la revendication 12, dans lequel le marqueur est une combinaison du marqueur individuel lié au fragment et du marqueur devant être incorporé dans le brin étendu pendant la réaction d'extension, et le marqueur individuel et le marqueur incorporé sont un double marqueur interactif contenant une paire d'un accepteur et d'un donneur.

15. Procédé selon la revendication 12, dans lequel l'hybridation de l'étape (e) est réalisée en présence d'un colorant intercalant, le marqueur contenu dans le brin étendu est un accepteur pour recevoir un signal du colorant intercalant, le signal du colorant intercalant intercalé dans un hybride entre le brin étendu et l'IO est transféré à l'accepteur, et l'accepteur fournit le signal détectable.

16. Procédé selon la revendication 1, dans lequel le PTO, le CTO et/ou l'IO est bloqué à son extrémité 3' pour empêcher son extension.

17. Procédé selon la revendication 1, dans lequel l'étape (f) de détection du signal est réalisée en détectant un signal sur le substrat solide fourni dans un processus de fusion d'un hybride entre le brin étendu et l'IO sur une plage de températures, ou de fusion et d'hybridation de l'hybride.

18. Procédé selon la revendication 1, dans lequel le procédé comprend en outre la répétition de l'intégralité ou de certaines des étapes (a)-(f) avec une dénaturation entre les cycles de répétition.

19. Procédé selon la revendication 1, dans lequel les étapes (a)-(f) sont réalisées dans un seul récipient de réaction ou dans des récipients de réaction distincts.

20. Procédé selon la revendication 1, dans lequel le procédé est réalisé pour détecter au moins deux types de séquences d'acide nucléique cible ; dans lequel l'oligonucléotide en amont comprend au moins deux types d'oligonucléotides, le PTO comprend au moins deux types des PTO, le CTO comprend au moins deux types des CTO et l'IO comprend au moins deux types des IO.

21. Procédé selon la revendication 1, dans lequel la séquence d'acide nucléique cible comprend une variation de nucléotide.

22. Procédé selon l'une quelconque des revendications 1 à 21, dans lequel le procédé est réalisé en présence d'une amorce en aval.

23. Kit de détection d'une séquence d'acide nucléique cible dans un échantillon d'acide nucléique par un essai de PCE-IH (hybridation d'oligonucléotide immobilisé par clivage et extension de PTO) sur une phase solide, comprenant :
(a) un oligonucléotide en amont ; dans lequel l'oligonucléotide en amont comprend une séquence de nucléotides s'hybridant complémentaire à la séquence d'acide nucléique cible ;
(b) un PTO (oligonucléotide de sondage et de marquage) ; dans lequel le PTO comprend (i) une partie de ciblage en 3' comprenant une séquence de nucléotides s'hybridant complémentaire à la séquence d'acide nucléique cible et (ii) une partie de marquage en 5' comprenant une séquence de nucléotides non complémentaire à la séquence d'acide nucléique cible ; dans lequel la partie de ciblage en 3' est hybridée avec la séquence d'acide nucléique cible et la partie de marquage en 5' n'est pas hybridée avec la séquence d'acide nucléique cible ; l'oligonucléotide en amont est localisé en amont du PTO ; dans lequel l'oligonucléotide en amont ou son brin étendu induit le clivage du PTO par l'enzyme ayant l'activité nucléase en 5' de sorte que le clivage libère un fragment comprenant la partie de marquage en 5' ou une partie de la partie de marquage en 5' du PTO ;
(c) un CTO (oligonucléotide de capture et de matrice) ; dans lequel le CTO comprend, dans une direction 3' vers 5', (i) une partie de capture comprenant une séquence de nucléotides complémentaire à la partie de marquage en 5' ou à une partie de la partie de marquage en 5' du PTO et (ii) une partie de matrice comprenant une séquence de nucléotides non complémentaire à la partie de marquage en 5' et à la partie de ciblage en 3' du PTO ; dans lequel le fragment libéré du PTO est hybridé avec la partie de capture du CTO ; dans lequel le fragment hybridé avec la partie de capture du CTO est étendu pour produire un brin étendu contenant une séquence étendue complémentaire à la partie de matrice du CTO et un duplex étendu est formé ; dans lequel le brin étendu comprend un marqueur ; et
(d) un IO (oligonucléotide immobilisé) ; dans lequel l'IO est immobilisé sur un substrat solide ; dans lequel l'IO comprend une séquence complémentaire au brin étendu ; dans lequel l'hybridation entre le brin étendu avec l'IO fournit un signal détectable sur le substrat solide ; dans lequel la séquence de l'IO est sélectionnée pour ne pas former d'hybride avec un PTO non digéré.

24. Kit selon la revendication 23, dans lequel le PTO et le CTO sont contenus selon un rapport molaire du PTO sur le CTO supérieur à 1.
